# EUROPÄISCHE PATENTSCHRIFT

(11) **EP 2 125 944 B1**
(45) Veröffentlichungstag und Bekanntmachung des Hinweises auf die Patenterteilung: **17.08.2011**
(21) Anmeldenummer: 07857378.9
(22) Anmeldetag: 11.12.2007
(51) Int. Cl.: C08K 5/17, C08F 220/00, F28D 1/00

(54) **VERFAHREN ZUR ÜBERTRAGUNG VON WÄRME AUF EIN WENIGSTENS EIN (METH)ACRYLMONOMERES ENTHALTENDES FLÜSSIGES GEMISCH**
PROCESS FOR TRANSFERRING HEAT TO A LIQUID MIXTURE COMPRISING AT LEAST ONE (METH)ACRYLIC MONOMER
PROCÉDÉ DE TRANSFERT DE CHALEUR DANS UN MÉLANGE LIQUIDE CONTENANT AU MOINS UN MONOMÈRE (MÉTH)ACRYLIQUE

(30) Priorität: 22.12.2006 DE 102006062258; 22.12.2006 US 871529 P
(43) Veröffentlichungstag der Anmeldung: 02.12.2009
(73) Patentinhaber: BASF SE, 67056 Ludwigshafen (DE)
(72) Erfinder: LIPOWSKY, Gunter, 69198 Schriesheim (DE); SCHLIEPHAKE, Volker, 67105 Schifferstadt (DE); RISSEL, Steffen, 67281 Kirchheim (DE); JÄGER, Ulrich, 67354 Römerberg (DE); HÖFER, Frank, 67098 Bad Dürkheim (DE); HAREMZA, Sylke, 69151 Neckargemünd (DE); ZUROWSKI, Peter, 76829 Landau (DE); MÜLLER-ENGEL, Klaus Joachim, 76297 Stutensee (DE)
(86) Internationale Anmeldenummer: PCT/EP2007/063686
(87) Internationale Veröffentlichungsnummer: WO 2008/077767

(56) Entgegenhaltungen:
- EP-A- 1 034 824
- DE-A1- 10 336 386
- GB-A- 1 282 854
- US-A1- 2005 074 563

## Beschreibung

Vorliegende Erfindung betrifft ein Verfahren zur Übertragung von Wärme auf ein wenigstens ein (Meth)acrylmonomeres enthaltendes flüssiges Gemisch mit Hilfe eines indirekten Wärmeaustauschers, der auf seiner Primärseite von einem fluiden Wärmeträger und auf seiner Sekundärseite gleichzeitig von dem wenigstens ein (Meth)acryl-monomeres enthaltenden flüssigen Gemisch durchströmt wird.

Die Schreibweise (Meth)acrylmonomere steht in dieser Schrift verkürzend für "Acrylmonomere und/oder Methacrylmonomere".

Der Begriff Acrylmonomere steht in dieser Schrift verkürzend für "Acrolein, Acrylsäure, Diacrylsäure, und/oder Ester der Acrylsäure".

Der Begriff Methacrylmonomere steht in dieser Schrift verkürzend für "Methacrolein, Methacylsäure und/oder Ester der Methacrylsäure".

(Meth)acrylmonomere sind wichtige Ausgangsverbindungen zur Herstellung von Polymerisaten, die z. B. als Klebstoff oder als Wasser superabsorbierende Materialien Verwendung finden.

Großtechnisch werden (Meth)acrolein und (Meth)acrylsäure überwiegend durch katalytische Gasphasenoxidation geeigneter C₃-/C₄-Vorläuferverbindungen (oder von Vorläuferverbindungen derselben), insbesondere von Propylen und Propan im Fall von Acrolein und Acrylsäure bzw. iso-Buten und iso-Butan im Fall der Methacryläsure und des Methacroleins, hergestellt. Neben Propylen, Propan, iso-Buten und iso-Butan eignen sich als Ausgangsstoffe auch andere 3 bzw. 4 Kohlenstoffatome enthaltende Verbindungen wie iso-Butanol, n-Propanol oder Vorläuferverbindungen derselben wie z. B. der Methylether von iso-Butanol.

(Meth)acrylsäure kann auch aus (Meth)acrolein (das z. B. durch Kondensation von Propionaldehyd und Formaldehyd erhältlich ist) erzeugt werden.

Dabei wird normalerweise ein Produktgasgemisch erhalten, aus dem die (Meth)acrylsäure bzw. das (Meth)acrolein abgetrennt werden muss.

Ester der (Meth)acrylsäure sind z. B. durch direkte Umsetzung von (Meth)acrylsäure und/oder (Meth)acrolein mit den entsprechenden Alkoholen erhältlich.

Allerdings fallen auch in diesem Fall zunächst Produktgemische an, aus denen die (Meth)acrylsäureester abgetrennt werden müssen.

Für vorstehende Abtrennungen werden häufig ein oder mehrere thermische Trennverfahren angewendet. Bei diesen wird in der Regel einem trennwirksamen Raum wenigstens ein wenigstens ein (Meth)acrylmonomeres enthaltender Stoffstrom zugeführt und wenigstens ein (davon verschiedener) wenigstens ein (Meth)acrylmonomeres enthaltender Stoffstrom aus demselben trennwirksamen Raum entnommen. Charakteristisch für die Mehrzahl thermischer Trennverfahren ist, dass die mit ihnen erzielte Trennwirkung in der Regel die Zufuhr von thermischer Energie (die Übertragung von Wärme) erfordert und dass an ihnen üblicherweise flüssige Phasen (flüssige Gemische) beteiligt sind (im trennwirksamen Raum geführt werden), die (Meth)acrylmonomere (wenigstens ein (Meth)acrylmonomeres) enthalten, (vgl. z. B. DE-A 10300816).
In der Regel wird diese thermische Energie (Wärme) mit Hilfe von indirekten Wärmeaustauschern zugeführt. Dazu wird aus einem Teil des trennwirksamen Raums häufig wenigstens ein wenigstens ein (Meth)acrylmonomeres enthaltendes flüssiges Gemisch entnommen. Dieses durchströmt dann (z. B. mit Hilfe von Pumpen und/oder durch natürlichen Umlauf) die Sekundärseite eines indirekten Wärmeaustauschers, der auf seiner Primärseite gleichzeitig von einem fluiden Wärmeträger durchströmt wird.
Aufgrund des zwischen den beiden Stoffströmen bestehenden Temperaturgradienten (der fluide Wärmeträger (darunter soll in dieser Schrift ein flüssiger Wärmeträger, ein gasförmiger Wärmeträger oder eine Gemisch aus einem flüssigen und einem gasförmigen (dampfförmigen) Wärmeträger verstanden werden) weist eine höhere Temperatur als das wenigstens ein (Meth)acrylmonomeres enthaltende flüssige Gemisch auf) findet ein Wärmeaustausch durch die die Primärseite von der Sekundärseite des indirekten Wärmeaustauschers trennende feste materielle Trennwand hindurch statt (d. h., ein indirekter Wärmeaustauscher soll in dieser Schrift ein Wärmeaustauscher sein, bei dem Wärmeträger und zu erwärmendes Gemisch nicht in materiellen Kontakt miteinander treten, sondern durch eine materielle Trennwand voneinander räumlich getrennt sind), aus dem eine Erwärmung des die Sekundärseite durchströmenden, wenigstens ein (Meth)acrylmonomeres enthaltenden, flüssigen Gemischs resultiert. Anschließend wird das die Sekundärseite des indirekten Wärmeaustauschers erwärmt verlassende, wenigstens ein (Meth)acrylmonomeres enthaltende flüssige Gemisch (dieses kann beim Wärmeaustausch auch teilweise oder vollständig in die Dampfphase überführt werden; in diesem Fall wird der Wärmeaustauscher häufig auch als Verdampfer bezeichnet; die Begriffe "gasförmig" und "dampfförmig" werden in dieser Schrift synonym verwendet) wenigstens teilweise in denselben Teil des trennwirksamen Raumes, dem es entnommen wurde und/oder in einen anderen Teil dieses trennwirksamen Raumes, rückgeführt, wobei die Entnahme- und die Rückführstelle räumlich auseinander liegen können (vgl. z. B. DE-A 10332758).

Nachteilig an einer wie vorstehend beschriebenen Verfahrensweise ist jedoch, dass (Meth)acrylmonomere, u. a. unter dem Einfluss erhöhter Temperatur und insbesondere in kondensierter Phase befindlich, in unerwünschter Weise zur Ausbildung von hochmolekularen Verbindungen (Polymerisaten) unterschiedlichster Art (radikalische Polymerisate, Polykondensate (z. B. Michael-Addukte) etc.) neigen. Durch den Zusatz sogenannter Polymerisationsinhibitoren (als solche kommen auch Stickstoff enthaltende Verbindungen in Betracht, die am Stickstoff wenigstens eine Phenylgruppe tragen (z. B. N,N'-Diisobutyl-para-phenylendiamin (Kerobit^{®} BTD), N,N-Diphenylamin, Methylenblau, oder Phenothiazin, vgl. z. B. EP-A 1 062 197, DE-A 103 36 386 und DE-A 102 35 847 sowie DE-A 29 01 783), die eine unerwünschte radikalische Polymerisation initiierende, sich zufällig ausbildende, Radikale in gewissem Umfang zu binden vermögen) lässt sich vorerwähnte unerwünschte Ausbildung hochmolekularer Verbindungen in beschränktem Umfang, aber leider nicht vollständig (vgl. DE-A 102 11 273) unterbinden, weshalb es auch bei Mitverwendung von radikalischen Polymerisationsinhibitoren insbesondere auf der der Sekundärseite zugewandten Fläche der materiellen Trennwand des indirekten Wärmeaustauschers im Verlauf einer wie beschrieben durchzuführenden indirekten Wärmeübertragung auf das wenigstens ein (Meth)acrylmonomeres enthaltende flüssige Gemisch im Lauf der Zeit zur Abscheidung von Feststoffen kommt (in der Literatur vielfach auch als Fouling bezeichnet), die den Wärmeübergang von der Primärseite auf die Sekundärseite mindert (vgl. US-A 3,271,296). Zu dieser Feststoffabscheidung tragen u. a. auch pyrolytische Zersetzungsreaktionen (bis zur Verkokung) bei. Von Zeit zu Zeit muss daher das thermische Trennverfahren unterbrochen werden, um die materielle Trennwand des indirekten Wärmeaustauschers, vor allem auf dessen Sekundärseite, zu reinigen.

Aus dem Stand der Technik sind nun unterschiedliche Maßnahmen bekannt, um das Fouling auf der Sekundärseite der indirekten Wärmeaustauscher zu mindern bzw. zeitlich zu verzögern.

Die US-A 3,271,296 empfiehlt zur Minderung der Ausbildung von Fouling den Zusatz von Reaktionsprodukten des Propylendiamin mit alkyl- und alkenylsubstituierten succinischen Carbonsäuren, denen dispergierende Wirkung zugeschrieben wird (z. B. Ko-mad^{®} 313 der Fa. Mol (Ungarn)). In entsprechender Weise empfiehlt die EP-A 1062197 den Zusatz von Tensiden zum zu erwärmenden, wenigstens ein (Meth)acrylmonomeres enthaltenden flüssigen Gemisch. Die EP-A 854129 empfiehlt zur Minderung des vorbeschriebenen Fouling grundsätzlich die Anwendung von Zwangsumlaufentspannungsverdampfern.

Nachteilig an den Empfehlungen des Standes der Technik ist jedoch, dass die Wirkung der empfohlenen Additive nicht im vollen Umfang zu befriedigen vermag. Dies gilt in gleicher Weise für die bloße Anwendung von Zwangsumlaufentspannungsverdampfern.

Die Aufgabe der vorliegenden Erfindung bestand daher darin, ein verbessertes Verfahren zur Übertragung von Wärme auf ein wenigstens ein (Meth)acrylmonomeres enthaltendes flüssiges Gemisch mit Hilfe eines indirekten Wärmeaustauschers zur Verfügung zu stellen.

Demgemäß wird ein Verfahren zur Übertragung von Wärme auf ein wenigstens ein (Meth)acrylmonomeres enthaltendes flüssiges Gemisch mit Hilfe eines indirekten Wärmeaustauschers, der auf seiner Primärseite von einem fluiden Wärmeträger und auf seiner Sekundärseite gleichzeitig von dem wenigstens ein (Meth)acrylmonomeres enthaltenden flüssigen Gemisch (stetig) durchströmt wird, gefunden, das dadurch gekennzeichnet ist, dass das wenigstens ein (Meth)acrylmonomeres enthaltende flüssige Gemisch wenigstens eine von (Meth)acrylmonomeren verschiedene Wirkverbindung aus der Gruppe bestehend aus tertiären Aminen, den Salzen gebildet aus einem tertiären Amin und einer Brönsted-Säure, sowie quartären Ammoniumverbindungen (den Salzen quartärer Ammoniumionen) mit der Maßgabe zugesetzt enthält, dass von den tertiären und quartären Stickstoffatomen in der wenigstens einen Wirkverbindung keines eine Phenylgruppe, aber wenigstens eine Teilmenge (wenigstens eines) wenigstens eine Alkylgruppe trägt.

Der Begriff (Meth)acrylmonomere soll, wie bereits gesagt, insbesondere die Verbindungen Acrolein, Methacrolein, Methacrylsäure und Acrylsäure umfassen. Aber auch die Diacrylsäure (olefinisch ungesättigtes Michael-Addukt zweier Acrylsäuremoleküle) und die Ester aus Acrylsäure bzw. Methacrylsäure und (vorzugsweise einwertigen (eine Hydroxylgruppe aufweisenden)) Alkoholen sollen unter diesem Begriff subsummieren. D. h., insbesondere auch die Ester der Acrylsäure mit (vorzugsweise einwertigen (eine Hydroxylgruppe aufweisenden)) C₁- bis C₈-Alkanolen (vor allem mit den C₄- bis C₈-Alkanolen) und die Ester der Methacrylsäure mit (vorzugsweise einwertigen (eine Hydroxylgruppe aufweisenden)) C₁- bis C₈-Alkanolen (vor allem mit den C₄- bis C₈-Alkanolen) sind (Meth)acrylmonomere im Sinn dieser Schrift. D. h., (Meth)acrylmonomere sind z. B. die nachfolgenden (Meth)acrylsäureester: Hydroxyethylacrylat, Hydroxyethylemethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, iso-Butylmethacrylat, n-Butylmethacrylat, tert.-Butylacrylat, tert.-Butylmethacrylat, Ethylacrylat, Ethylmethacrylat, 2-Ethylhexylacrylat und 2-Ethylhexylmethacrylat.

Unter einem tertiären Amin soll in dieser Schrift eine organische Verbindung verstanden werden, die wenigstens ein Stickstoffatom chemisch gebunden enthält, das zu drei voneinander verschiedenen Kohlenstoffatomen (zu nicht mehr und nicht zu weniger als diesen drei und auch zu keinem anderen Atom) mit der Maßgabe eine chemische Bindung (in der Regel ein kovalente) aufweist, dass keines der drei Kohlenstoffatome gleichzeitig eine chemische Doppelbindung zu einem Sauerstoffatom oder einem anderen Element der Sauerstoff enthaltenden Gruppe im Periodensystem der Elemente aufweist. Ein solches Stickstoffatom wird in dieser Schrift als tertiäres Stickstoffatom bezeichnet.

Unter einer quartären Ammoniumverbindung soll in dieser Schrift eine ionische Verbindung (das Salz eines quartären Ammoniumions) verstanden werden, die wenigstens ein Stickstoffatom enthält, das zu vier voneinander verschiedenen Kohlenstoffatomen (und zu sonst keinen anderen Atomen) mit der Maßgabe eine chemische Bindung (in der Regel eine kovalente) aufweist, dass keines der vier Kohlenstoffatome gleichzeitig eine chemische Doppelbindung zu einem Sauerstoffatom oder einem anderen Element der Sauerstoff enthaltenden Gruppe im Periodensystem der Elemente aufweist. Ein solches Stickstoffatom wird in dieser Schrift als ein quartäres Stickstoffatom bezeichnet. Das es enthaltende positiv geladene Ion wird in dieser Schrift als quartäres Ammoniumion bezeichnet.

Unter einer Phenylgruppe soll in dieser Schrift jeder aus sechs C-Atomen bestehende aromatische Ring (charakteristisches Merkmal eines solchen aromatischen Ringsystems ist, dass die sechs C-Atome im Röntgenstrahlbeugungsexperiment in einer Ebene liegen) verstanden werden, unabhängig davon, ob diese sechs C-Atome mit einem Wasserstoffatom oder mit einem Substituenten für das Wasserstoffatom chemisch verbunden sind. Ein tertiäres oder quartäres Stickstoffatom trägt dann eine Phenylgruppe, wenn es an eines der sechs C-Atome des aromatischen Rings chemisch (kovalent) gebunden ist. D. h., Triphenylamin, Chinolin, Phenothiazin und Methylenblau sind keine Wirkverbindung im Sinne dieser Erfindung.

Selbstverständlich kann eine erfindungsgemäß zuzusetzende Wirkverbindung mehr als ein tertiäres Stickstoffatom oder mehr als ein quartäres Stickstoffatom aufweisen. Natürlich kann eine erfindungsgemäß zuzusetzende Wirkverbindung auch wenigstens ein tertiäres Stickstoffatom und wenigstens ein quartäres Stickstoffatom gleichzeitig aufweisen.

Relativ zu einem bestimmten tertiären Amin ist eine Brönsted-Säure eine solche chemische Verbindung, die an das tertiäre Amin ein Proton abzugeben vermag, wodurch das tertiäre Amin in ein nicht quartäres, elektrisch positiv geladenes, Ammoniumion übergeht (vgl. Grundlagen der organischen Chemie; Hans Rudolf Christen; Verlag Sauerländer Aarau, Diesterweg•Salle Frankfurt am Main, 1975, S. 383 und folgende). Die Brönsted-Säure geht dabei ihrerseits gleichzeitig in ihre konjugierte anionische Base über.

Erfindungsgemäß besonders geeignete Wirkverbindungen sind tertiäre aliphatische Amine und deren nicht quartäre Ammoniumsalze. Diese Amine können dadurch vom Ammoniak abgeleitet werden, dass man dessen drei H-Atome durch drei Alkylgruppen ersetzt.

Unter diesen tertiären aliphatischen Aminen sind jene der allgemeinen Formel I (sowie die daraus resultierenden nicht quartären Ammoniumsalze) bevorzugt: 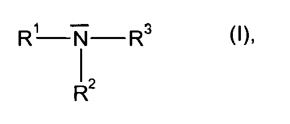 mit R¹, R² und R³ unabhängig voneinander eine 1 bis 8 C-Atome, vorzugsweise 1 bis 6 C-Atome und besonders bevorzugt 1 bis 4 C-Atome aufweisende Alkylgruppe (diese Gruppen sind nur aus Wasserstoff- und Kohlenstoffatomen aufgebaut und umfassen nicht die Cycloalkylgruppen) oder Cycloalkylgruppe (sind ebenfalls nur aus Wasserstoff und Kohlenstoff aufgebaut).

Unter den tertiären aliphatischen Aminen der allgemeinen Formel I sind wiederum diejenigen besonders bevorzugt, in denen R¹ = R² = R³ (und vorzugsweise eine Alkylgruppe) zutrifft (sowie die daraus resultierenden nicht quartären Ammoniumsalze (d. h., ihre Salze mit einer Brönsted-Säure).

Bevorzugte Reste R¹, R², R³ sind die Methylgruppe, die Ethylgruppe, die iso-Propylgruppe, die n-Propylgruppe, die n-Butylgruppe, die tert.-Butylgruppe und die n-Hexylgruppe sowie die Cyclohexylgruppe.

D. h., erfindungsgemäß besonders vorteilhafte Wirkverbindungen sind z. B. Trimethylamin, Triethylamin, Tri-n-Hexylamin, Tri-n-Butylamin und N-Ethyl-N,N-diisopropylamin (sowie deren Salze mit einer Brönsted-Säure).

Ferner kommen als erfindungsgemäße Wirkverbindungen solche in Betracht (im weiteren Verlauf dieser Schrift als Wirkverbindungen der allgemeinen Formel II bezeichnet), die sich von den Wirkverbindungen der allgemeinen Formel (I) formal dadurch ableiten, dass wenigstens einer der Rest R¹, R², R³ eine Alkyl- oder eine Cycloalkylgruppe ist, bei der ein oder mehr Wasserstoffatome durch wenigstens eine der Gruppen -OH, -NH₂, -NHCH₃ und -N(CH₃)₂ ersetzt und/oder die (gegebenenfalls cyclische) Kohlenstoffkette wenigstens einmal durch ein Sauerstoffatom unterbrochen ist (sowie deren Salze mit einer Brönsted-Säure). Unter diesen seien besonders hervorgehoben das N,N,N',N'-Tetramethyl-1,3-propandiamin, das N,N-Diethylethanolamin, sowie Bis (2-dimethylaminoethyl)ether, Pentamethyldiethylentriamin, 3-Dimethylamino-1-propylamin, N,N,N',N'-Tetramethyl-1,6-hexandiamin und N,N-Dimethylcyclohexylamin (sowie deren Salze mit einer Brönsted-Säure).

Als weitere erfindungsgemäß günstige tertiäre Amine (Wirkverbindungen III) kommen Derivate des 1,3-Diazols (Imidazols) in Betracht, bei denen der Wasserstoff am Stickstoff des 1,3-Diazols in der 1-Stellung durch eine Alkylgruppe R⁴ mit 1 bis 8 C-Atomen, vorzugsweise 1 bis 6 C-Atomen und besonders bevorzugt 1 bis 4 C-Atomen ersetzt und/oder der Stickstoff des 1,3-Diazols in der 3-Stellung mit einer Alkylgruppe R⁵ mit 1 bis 8 C-Atomen, vorzugsweise 1 bis 6 C-Atomen und besonders bevorzugt 1 bis 4 C-Atomen alkyliert ist. Beim Fall der "und"-Verknüpfung handelt es sich um die sogenannten Imidazoliumsalze, deren Kation im Sinne der Erfindung zwei tertiäre Stickstoffatome aufweist. Bei der "oder"-Fallgestaltung handelt es sich um Imidazoliumsalze mit im Sinne der Erfindung nur einem tertiären Stickstoffatom.

Beispiele für derartige erfindungsgemäß geeignete tertiäre Amine sind das 1-Methylimidazol, das 1-Ethylimidazol, das 1-Propylimidazol, das 1-Ethyl-3-methyl-imidazoliumacrylat, das 1-Ethyl-3-methylimidazoliumacetat, das 1-n-Butyl-3-methylimidazoliumacetat und das 1-Ethyl-3-methylimidazoliumchlorid. Erfindungsgemäß bevorzugt sind unter den vorgenannten Imidazoliumsalzen jene, deren Anion das Hydroxid (^{⊝}OH), oder die anionische konjugierte Base einer einwertigen Brönsted-Säure (vermag an eine starke Base wie Natronlauge nur eine Proton abzugeben; d. h., Schwefelsäure ist eine zweiwertige Brönsted-Säure) ist. Zu diesen einfach negativ geladenen Anionen zählen insbesondere die Carboxylatanionen von einwertigen organischen Carbonsäuren wie z. B. Ameisensäure, Essigsäure, Propionsäure, Acrylsäure, Methacrylsäure, Chloressigsäure und Nitroessigsäure. Insbesondere zählen zu diesen einfach negativ geladenen Anionen aber auch die konjugierten Basen von starken einwertigen anorganischen Brönsted-Säuren wie HCl, HBr, HJ, HNO₃ und HClO₃.

Die vorgenannten Brönsted-Säuren (einschließlich Wasser) sind auch diejenigen, die besonders geeignet sind, um erfindungsgemäß geeignete tertiäre Amine (insbesondere die tertiären Amine (I) und (II)) in erfindungsgemäß geeignete Salze derselben (nicht quartäre Ammoniumverbindungen) zu überführen.

Eine weitere Gruppe erfindungsgemäß geeigneter Wirkverbindungen sind quartäre Ammoniumverbindungen. Zu diesen zählen vor allem die Salze aus quartären Ammonium(kat)ionen und den anionischen konjugierten Basen von Brönsted-Säuren (z. B. die Salze der DE-A 10314203), insbesondere von einwertigen Brönsted-Säuren. Zu diesen bevorzugten einfach negativ geladenen Anionen zählen insbesondere das ^{⊝}OH, die Carboxylationen von einwertigen organischen Carbonsäuren wie z. B. Ameisensäure, Essigsäure, Propionsäure, Acrylsäure, Methacrylsäure, Chloressigsäure und Nitroessigsäure. Insbesondere zählen zu diesen einfach negativ geladenen Anionen aber auch die konjugierten Basen von einwertigen anorganischen Brönsted-Säuren. Solche Anionen sind z. B. das Cl^{⊝}, Br^{⊝}, J^{⊝}, NO₃^{⊝} und ClO₃^{⊝}.

Bevorzugte quartäre Ammonium(kat)ionen sind vor allem diejenigen, die aus den Wirkverbindungen (I) und (II) formal dadurch erhältlich sind, dass man an deren tertiäres Stickstoffatom eine weitere 1 bis 8 C-Atome, vorzugsweise 1 bis 6 C-Atome und besonders bevorzugt 1 bis 4 C-Atome aufweisende Alkylgruppe R⁶ bindet.

Derartige quartäre Ammoniumionen subsumieren unter die allgemeine Formel (IV), 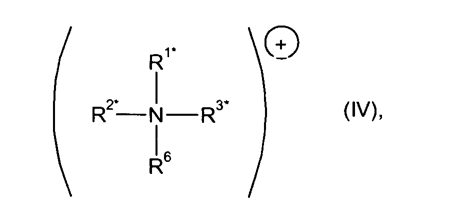
- mit R¹*, R²* und R³*: unabhängig voneinander und unabhängig von R⁶ eine 1 bis 8 C-Atome, vorzugsweise 1 bis 6 C-Atome und besonders bevorzugt 1 bis 4 C-Atome aufweisende Alkylgruppe oder Cycloalkylgruppe, oder eine der vorgenannten Alkyl- oder Cycloalkylgruppen, bei der ein oder mehr Wasserstoffatome durch wenigstens eine der Gruppen -OH, -NH₂, -NHCH₃ und -N(CH₃)₂ ersetzt und/oder die (gegebenenfalls cycli- sche) Kohlenstoffkette wenigstens einmal durch ein Sauer- stoffatom unterbrochen ist.

Besonders bevorzugte quartäre Ammoniumionen IV sind diejenigen der allgemeinen Formel (V), 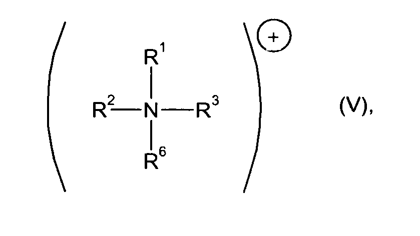 in der R¹, R², R³ und R⁶ unabhängig voneinander die in dieser Schrift diesen Resten zugeschriebene Bedeutung aufweisen können.

Besonders bevorzugte quartäre Ammoniumionen (V) sind diejenigen mit R¹ = R² = R³ = R⁶ (und vorzugsweise eine Alkylgruppe).

Beispielhaft erwähnt seien als erfindungsgemäß ganz besonders bevorzugte quartäre Ammoniumverbindungen das Tetramethylammoniumacetat, das Tetramethylammoniumchlorid, das Tetramethylammoniumhydroxid und das Tetramethylammoniumacrylat.

Grundsätzlich können alle Reste R¹, R², R³, R⁴, R⁵, R⁶ sowie R¹*, R²* und R³*, für den Fall, dass sie nicht cyclisch sind, sowohl geradkettig oder verzweigt sein.

In der Regel wird man für das erfindungsgemäße Verfahren dem wenigstens ein (Meth)acrylmonomeres enthaltenden flüssigen Gemisch wenigstens 0,01, bzw. wenigstens 0,05 Gew.-%, oft 0,1 bis 10 Gew.-%, häufig 0,1 bis 5 Gew.-%, vielfach 0,1 bis 3 Gew.-% und bevorzugt 0,5 bis 2 Gew.-% bzw. 0,5 bis 1 Gew.-% (jeweils bezogen auf sein Gewicht) wenigstens einer erfindungsgemäßen Wirkverbindung zusetzen.

Erfindungsgemäß vorteilhaft sind die erfindungsgemäß zuzusetzenden Wirkverbindungen so beschaffen, dass sie sich unter den Einsatzbedingungen des erfindungsgemäßen Verfahrens (Arbeitsdruck, Arbeitstemperatur) in der jeweiligen Zusatzmenge in dem wenigstens ein (Meth)acrylmonomeres enthaltenden flüssigen Gemisch vollständig lösen.

Vor diesem Hintergrund sind solche erfindungsgemäß zuzusetzenden Wirkverbindungen bevorzugt, deren Molgewicht ≤ 600 g, besser ≤ 500 g, mit Vorteil ≤ 400 g, in günstiger Weise ≤ 300 g, vorzugsweise ≤ 250 g, besonders bevorzugt ≤ 200 g und ganz besonders bevorzugt ≤ 180 g beträgt. Normalerweise wird das Molgewicht erfindungsgemäß zuzusetzender Wirkverbindungen jedoch ≥ 59,1 g betragen.

Die erfindungsgemäß zu erwärmenden flüssigen Gemische können das wenigstens eine (Meth)acrylmonomere sowohl in mehr oder weniger reiner Form als auch in Verdünnung befindlich (z. B. mit Lösungsmitteln) enthalten. Das Lösungsmittel kann dabei sowohl ein wässriges als auch ein organisches Lösungsmittel sein. Das heißt, ein erfindungsgemäß zu erwärmendes flüssiges Gemisch kann (bezogen auf sein Gewicht) z. B. zu ≥ 0,05 Gew.-%, oder ≥ 0,1 Gew.-%, oder ≥ 0,5 Gew.-%, oder ≥ 1 Gew.-%, oder ≥ 1,5 Gew.-%, oder ≥ 2 Gew.-%, oder ≥ 10 Gew.-%, oder ≥ 20 Gew.-%, oder ≥ 40 Gew.-%, oder ≥ 60 Gew.-%, oder ≥ 80 Gew.-%, oder ≥ 90 Gew.-%, oder ≥ 95 Gew.-%, oder ≥ 99 Gew.-% von dem wenigstens einen (Meth)acrylmonomeren enthalten. Selbstredend liegt der Gehalt an einem bestimmten (Meth)acrylmonomeren stets bei Werten < 100 Gew.-% (Vorstehendes ist gleichzeitig die Definition des Begriffes "wenigstens ein (Meth)acrylmonomeres enthaltendes flüssiges Gemisch", wie er in dieser Schrift verwendet wird; er meint eine flüssige Substanz, die neben wenigstens einem bestimmten (Meth)acrylmonomeren noch wenigstens ein zweites (Meth)acryl-monomeres und/oder noch wenigstens eine von einem (Meth)acrylmonomeren verschiedene zweite Substanz enthält; die Mengenanteile können dabei völlig beliebig sein, so lange sie nur analytisch (z. B. gaschromatographisch oder mittels HPLC) nachweisbar sind; beispielsweise kann die zweite von einem (Meth)acrylmonomeren verschiedene Substanz ein Polymerisationsinhibitor sein.

An dieser Stelle sei festgehalten, dass alle in dieser Schrift gemachten Aussagen insbesondere dann gültig sind, wenn das wenigstens eine (Meth)acrylmonomere Acrylsäure ist. Der Offenbarungsgehalt dieser Schrift soll daher insbesondere auch diejenigen Aussagen umfassen, die dadurch entstehen, dass man dort, wo in dieser Schrift "(Meth)acrylmonomere(s)" steht, diesen Begriff durch "Acrylsäure" ersetzt.

Die Temperatur, mit der das wenigstens ein (Meth)acrylmonomeres enthaltende flüssige Gemisch den indirekten Wärmeaustauscher (d. h., nach der erfolgten erfindungsgemäßen Übertragung von Wärme) wieder verlässt (T^{aus}), wird in der Regel 50 bis 350 °C, oder 100 bis 300 °C, häufig 150 bis 250 °C, und oft 170 bis 220 °C betragen. Der Druck innerhalb des indirekten Wärmaustauschers kann sowohl bei Atmosphärendruck (1 atm) als auch oberhalb oder unterhalb von Atmosphärendruck liegen. Typische für die erfindungsgemäße Wärmeübertragung geeignete Druckbereiche sind 1 mbar bis 10 bar, oft 10 mbar bis 5 bar und vielfach 50 mbar bis 3 bar. Die Temperatur, mit der das wenigstens ein (Meth)acrylmonomeres enthaltende flüssige Gemisch beim erfindungsgemäßen Verfahren in den indirekten Wärmeaustauscher eintritt, um in diesem erwärmt zu werden (T^{ein}), kann z. B. 50 bis 350 °C, häufig 70 bis 250 °C, und vielfach 120 bis 220 °C betragen. Der Unterschied zwischen T^{ein} und T^{aus}, d. h., die Differenz T^{aus} - T^{ein}, wird in der Regel 0,1 bis 50 °C, häufig 0,5 bis 25 °C, und vielfach 1 bis 10 °C betragen. Der Arbeitsdruck des wenigstens ein (Meth)acrylmonomeres enthaltenden flüssigen Gemischs ist beim Eintritt desselben in den indirekten Wärmeaustauscher größer als beim Austritt aus dem wenigstens einen Wärmeaustauscher. Grundsätzlich kann das wenigstens ein (Meth)acrylmonomeres enthaltende flüssige Gemisch bei seinem Austritt aus dem indirekten Wärmeaustauscher vollständig in die Dampfphase (Gasphase) überführt sein.

Im übrigen enthält das wenigstens ein (Meth)acrylmonomeres enthaltende flüssige Gemisch in der Regel auch Polymerisationsinhibitoren gegen radikalische Polymerisation zugesetzt. Als solche kommen grundsätzlich alle diejenigen in Betracht, die im Stand der Technik zum Zweck der Inhibierung einer radikalischen Polymerisation von in flüssiger Phase befindlichen (Meth)acrylmonomeren empfohlen werden. Als solche Polymerisationsinhibitoren kommen in Betracht Alkylphenole, beispielsweise o-, m-oder p-Kresol (Methylphenol), 2-tert.-Butyl-4-methylphenol, 6-tert.-Butyl-2,4-dimethylphenol, 2,6-Di-tert.-Butyl-4-methylphenol, 2-tert.-Butylphenol, 4-tert.Butylphenol, 2,4-di-tert.-Butylphenol, 2-Methyl-4-tert.-Butylphenol, 4-tert.-Butyl-2,6-dimethylphenol, oder 2,2'-Methylen-bis-(6-tert.-butyl-4-methylphenol). Hydroxyphenole, beispielsweise Hydrochinon, 2-Methylhydrochinon, 2,5-Di-tert.-Butylhydrochinon, Brenzcatechin (1,2-Dihydroxybenzol) oder Benzochinon, Aminophenole wie z. B. para-Aminophenol, Nitrosophenole wie z. B. para-Nitrosophenol, Alkoxyphenole, beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol, Tocopherole wie z. B. o-Tocopherol sowie 2,3-Dihydro-2,2-dimethyl-7-hydroxybenzofuran (2,2-Dimethyl-7-hydroxycumaran), N-Oxyle wie 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-Tetramethyl-piperidin-N-oxyl, 4,4',4"-Tris (2,2,6,6-tetramethyl-piperidin-N-oxyl)-phosphit oder 3-Oxo-2,2,5,5-tetramethyl-pyrrolidin-N-oxyl, aromatische Amine oder Phenylendiamine wie z. B. N,N-Diphenyl-amin, N-Nitroso-di-phenylamin und N,N'-Dialkyl-para-phenylendiamin, wobei die Alkylreste gleich oder verschieden sein können und jeweils unabhängig voneinander aus 1 bis 4 Kohlenstoffatomen bestehen und gradkettig oder verzweigt sein können, Hydroxylamine wie z. B. N,N-Diethylhydroxylamin, phosphorhaltige Verbindungen wie z. B. Triphenylphosphin, Triphenylphosphit, hyposphosphorige Säure oder Triethylphosphit, schwefelhaltige Verbindungen wie z. B. Diphenylsulfid oder Phenothiazin, gegebenenfalls in Kombination mit Metallsalzen wie beispielsweise die Chloride, Dithiocarbamate, Sulfate, Salicylate oder Acetate von Kupfer, Mangan, Cer, Nickel oder Chrom. Selbstverständlich können auch unterschiedlichste Gemische der genannten Polymerisationsinhibitoren eingesetzt werden. Bevorzugt werden als Polymerisationsinhibitor Phenothiazin und/oder Hydrochinonmonomethylether eingesetzt. Vielfach werden die benannten Polymerisationsinhibitoren durch ein molekularen Sauerstoff enthaltendes Gas (z. B. Luft oder mit Stickstoff verdünnte Luft) unterstützt. Je nach verwendetem Polymerisationsinhibitor wird seine Einsatzmenge in dem wenigstens ein (Meth)acrylmonomeres enthaltenden flüssigen Gemisch 10 bis 1000 Gew.ppm, häufig 50 bis 500 Gew.ppm und vielfach 150 bis 350 Gew.ppm betragen (jeweils bezogen auf den Gesamtgehalt an (Meth)acrylmonomeren im flüssigen Gemisch).

Beim indirekten Wärmeaustauscher geschieht die Wärmeübertragung nicht im durch Mischen erzwungenen direkten Kontakt zwischen fluidem Wärmeträger und zu erwärmendem flüssigen Gemisch. Vielmehr erfolgt die Wärmeübertragung indirekt zwischen den durch eine Trennwand getrennten Fluiden. Die für den Wärmetransport aktive Trennfläche des Wärmeübertragers (Wärmeaustauschers) wird als Wärmeaustausch-oder Übertragerfläche bezeichnet, und der Wärmetransport folgt den bekannten Gesetzen des Wärmedurchgangs.

Erfindungsgemäß wesentlich ist, dass beim erfindungsgemäßen Verfahren der indirekte Wärmeaustauscher sowohl vom fluiden Wärmeträger als auch vom das wenigstens eine (Meth)acrylmonomere enthaltenden flüssigen Gemisch durchströmt wird. D. h., beide strömen in den Wärmeaustauscher hinein und anschließend wieder heraus.

Als fluide Wärmeübertrager kommen für das erfindungsgemäße Verfahren grundsätzlich alle möglichen heißen Gase, Dämpfe und Flüssigkeiten in Betracht. In erster Linie zählt dazu Wasserdampf, der sich auf unterschiedlichen Drücken und Temperaturen befinden kann. Häufig ist es günstig, wenn der Wasserdampf beim Durchströmen des indirekten Wärmeaustauschers kondensiert (Sattdampf). Alternativ kommen als fluide Wärmeträger Öle, Schmelzen, organische Flüssigkeiten sowie heiße Gase in Betracht. Beispiele dafür sind Siliconverbindungen wie Tetraarylsilikat, Diphenylgemisch aus 74 Gew.-% Diphenylether und 26 Gew.-% Diphenyl, chloriertes nichtbrennendes Diphenyl sowie Mineralöle und Druckwasser.

Erfindungsgemäß geeignete indirekte Wärmeaustauscher sind insbesondere Doppelrohr-, Rohrbündel-, Rippenrohr-, Spiral- oder Plattenwärmeübertrager. Doppelrohrwärmeübertrager bestehen aus zwei ineinanderliegenden Rohren. Mehrere dieser Doppelrohre können zu Rohrwänden verbunden werden. Das Innenrohr kann glatt oder zum Verbessern des Wärmeübergangs mit Rippen versehen sein. Auch kann in Einzelfällen ein Rohrbündel das Innenrohr vertreten. Die im Wärmeaustausch stehenden Fluide bewegen sich im Gleichstrom oder im Gegenstrom. Das wenigstens ein (Meth)acrylmonomeres enthaltende flüssige Gemisch strömt erfindungsgemäß zweckmäßig im Innenrohr aufwärts und z. B. heißer Wasserdampf im Ringraum abwärts. Dem angestrebten Durchsatz angepasste Rohrdurchmesser erteilen den fluiden Medien Strömungsgeschwindigkeiten, die hohe Wärmedurchgangszahlen ergeben.

Der Temperaturunterschied zwischen fluidem Wärmeträger und dem wenigstens ein (Meth)acrylmonomeres enthaltenden fluiden Gemisch kann innerhalb des indirekten Wärmeaustauschers ganz generell z. B. 5 °C bis 150 °C, häufig10 °C. bis 100 °C, oder 20 °C bis 80 °C betragen.

Rohrbündelwärmeübertrager bestehen normalerweise aus einem abgeschlossenen weiten Mantelrohr, das die an Rohrböden befestigten zahlreichen glatten oder gerippten Übertragerrohre kleinen Durchmessers umschließt. Der Abstand von Rohrmitte zu Rohrmitte der Bündelrohre beträgt anwendungstechnisch zweckmäßig das 1,3- bis 2,5-fache der Rohraußendurchmesser. Die entstehende große spezifische Wärmeaustauschfläche - als Austauschfläche je Einheit des Raumbedarfs - ist ein Vorzug des Rohrbündelwärmeübertragers. Die senkrecht oder waagrecht angeordneten Rohrbündelwärmeübertrager unterscheiden sich u.a. in der Rohrführung. Die Übertragerrohre können gerade, U-förmig gebogen oder auch als mehrgängiges Spiralrohrbündel ausgeführt sein. Das erfindungsgemäß zu erwärmende wenigstens ein (Meth)acrylmonomeres enthaltende flüssige Gemisch strömt erfindungsgemäß bevorzugt innerhalb der Übertragerrohre (grundsätzlich kann es aber auch im die Übertragerrohre umgebenden Raum und der Wärmeträger in den Übertragerrohren strömen). Der fluide Wärmeträger (vorzugsweise Wassersattdampf) strömt erfindungsgemäß zweckmäßig außerhalb der Übertragerrohre. Leitbleche zur besseren Führung des fluiden Wärmeträgers im Mantelraum sind erfindungsgemäß zweckmäßig und dienen in der Regel dem Zusatzzweck, die Übertragerrohre zu stützen. Die Leitbleche erhöhen in der Regel die Strömungsgeschwindigkeiten im Mantelraum und u. a. damit die Wärmeübergangszahlen. Die Strömung verläuft im Mantelraum mit Vorteil quer zu den Übertragerrohren. Nach der Strömungsrichtung des Mantelraumfluids in Bezug auf die Übertragerrohre sind z. B. Längsstrom- und Kreuzstrom- sowie Querstromrohrbündelwärmeübertrager unterscheidbar. Grundsätzlich kann der fluide Wärmeübertrager auch mäanderförmig um die Übertragerrohre bewegt und lediglich über den Rohrbündelwärmeaustauscher betrachtet im Gleich- oder Gegenstrom zum erfindungsgemäß zu erwärmenden flüssigen Gemisch geführt werden. Auch Spiralrohrbündel-Wärmeübertrager nutzen in der Regel die Vorteile des Kreuzstroms. Die Rohre wechseln - von Lage zu Lage - rechts-linksgängig ab. Das Mantelraumfluid fließt im Gegenstrom zum Rohrfluid und umströmt die Spiralrohre im Kreuzstrom.

Im Einstromrohrbündelwärmeübertrager bewegt sich das erfindungsgemäß zu erwärmende wenigstens ein (Meth)acrylmonomeres enthaltende flüssige Gemisch durch alle Übertragerrohre in der gleichen Richtung.

Mehrstromrohrbündelwärmeübertrager enthalten in einzelne Sektionen unterteilte Rohrbündel (in der Regel enthalten die einzelnen Sektionen eine identische Anzahl von Rohren). Trennwände teilen sich an die Rohrböden (durch die die Übertragerrohre abgedichtet geführt und an denen sie befestigt sind) anschließende Kammern in Abschnitte auf und lenken das aus einer Sektion in den Kammerteil eintretende flüssige, wenigstens ein (Meth)acrylmonomeres enthaltende Gemisch in eine zweite Sektion um und damit zurück. Das erfindungsgemäß zu erwärmende flüssige Gemisch durchfließt je nach Anzahl der Sektionen die Länge des Rohrbündelwärmeübertragers mehrmals (zweimal, dreimal, viermal etc.) mit hoher Geschwindigkeit in alternierender Richtung (Zweistrom-, Dreistrom-, Vierstrom- etc. -rohrbündelwärmeübertrager). Wärmeübergangszahl und Austauschweg nehmen entsprechend zu.

Plattenwärmeübertrager (Plattenwärmeaustauscher) sind normalerweise nach Art der Filterpressen in der Regel aus gewellten oder anderweitig profilierten, mit Kanälen für den fluiden Wärmeträger und das aufzuwärmende flüssige Gemisch versehenen Platten (in der Regel aus Graphit oder Metall, z. B. Edelstahl) in kompakter Bauweise zusammengesetzt. Die beiden Wärme austauschenden Fluide fließen dann im Gleich-, Gegen- und/oder Querstrom als dünne Schichten wechselnd (z. B. auf- und abwärts) durch ihre Kammerreihen und stehen an beiden Kammerwänden miteinander in Wärmeübertragung. Die gewellten Plattenprofile erhöhen die Turbulenz und verbessern die Wärmeübergangszahlen. Für den erfindungsgemäßen Zweck geeignete Plattenwärmeaustauscher sind z. B. beschrieben in der EP-A 107 9194, der US-A 6,382,313, der EP-A 123 2004 und der WO 01/32301. Rohrbündelwärmeaustauscher sind z.B. beschrieben in der EP-A 700 893, EP-A 700 714 und DE-A 443 1949. Spiral- und Rippenrohrwärmeaustauscher findet man z. B. beschrieben in Vauck/Müller, Grundoperationen chemischer Verfahrenstechnik, 4. Auflage, Verlag Theodor Steinkopf, Dresden (1974) sowie in Ullmanns Encyclopädie der technischen Chemie, Band 2, Verfahrenstechnik I (Grundoperationen), 4. Auflage, 1972, S. 432 ff.

Für das erfindungsgemäße Verfahren ganz besonders geeignete Wärmeaustauscher sind die in der EP-A 854 129 als Stand der Technik und als Erfindung beschriebenen (insbesondere die in den Figuren 1 bis 3 abgebildeten) Wärmeaustauscher. Dazu gehören insbesondere der Zwangsumlaufröhrenverdampfer, der Zwangsumlaufröhrenentspannungsverdampfer und der Robert-Verdampfer. Der Begriff Zwangsumlauf bedeutet in diesem Zusammenhang, dass das wenigstens ein (Meth)acrylmonomeres enthaltende flüssige Gemisch mit Hilfe einer Pumpe durch den indirekten Wärmeaustauscher (dessen Rohre) gefördert wird, während die Förderung im Rohrbündel-Robert-Verdampfer durch die aufsteigenden Siededampfblasen und den Dichteunterschied erfolgt. Die EP-A 854 129 bildet daher einen in diese Schrift integrierten Bestandteil.

Das erfindungsgemäße Verfahren eignet sich insbesondere für solche wenigstens ein (Meth)acrylmonomeres enthaltenden flüssigen Gemische, die im Rahmen eines thermischen Trennverfahrens zur Auftrennung eines wenigstens ein (Meth)acrylmono-meres enthaltenden Gemisches aus einem Teil eines trennwirksamen Raumes, dem wenigstens ein wenigstens ein (Meth)acrylmonomeres enthaltender Stoffstrom zugeführt und wenigstens ein von diesem verschiedener wenigstens ein (Meth)acryl-monomeres enthaltender Stoffstrom entnommen wird, entnommen, mit Hilfe von Pumpen und/oder durch natürlichen Umlauf durch die Sekundärseite des indirekten Wärmeaustauschers geführt (gefördert) und nach dem Verlassen des indirekten Wärmeaustauschers in flüssiger und/oder dampfförmiger Form wenigstens teilweise in denselben Teil des trennwirksamen Raumes, dem es entnommen wurde, und/oder in einen anderen Teil desselben trennwirksamen Raumes und/oder in einen Teil eines anderen trennwirksamen Raumes rück- bzw. zugeführt werden.

Häufig umfasst der trennwirksame Raum eine trennwirksame Einbauten enthaltende (grundsätzlich kann er aber auch eine an trennwirksamen Einbauten freie Trennkolonne umfassen) Trennkolonne (vgl. z. B. DE-A 10300816). Die im trennwirksamen Raum, z. B. in der Trennkolonne, mitverwendeten trennwirksamen Einbauten verfolgen den Zweck, die Oberfläche für den Wärme- und Stoffaustausch im trennwirksamen Raum, der im Rahmen eines thermischen Trennverfahrens die Auftrennung bewirkt, zu erhöhen. Als solche Einbauten kommen z. B. Packungen, Füllkörperschüttungen und/oder Stoffaustauschböden jedweder Art (vgl. z. B. alle in der DE-A 10336386 individuell genannten Einbauten) in Betracht.

Beispiele für solche thermischen Trennverfahren sind die Rektifikation, die Desorption, die Strippung, die Destillation, die azeotrope Rektifikation und die Überlagerung von Absorption und Rektifikation. Bei all diesen Verfahren handelt es sich um solche, bei denen in die trennwirksame Einbauten enthaltenden Trennkolonnen gasförmige (aufsteigend) und flüssige (absteigend) Stoffströme im Gegenstrom geführt werden, wobei infolge der zwischen den Stoffströmen bestehenden Gradienten ein Wärme- und Stoffaustausch stattfindet, der letztlich die in der Trennkolonne gewünschte Trennung bedingt. Beim thermischen Trennverfahren der Strippung (ein Strippgas nimmt aus einer Flüssigkeit in selbiger gelöst enthaltende Komponenten mit unterschiedlicher Affinität auf) und der Desorption (der Umkehrprozess zur Absorption; das in der Flüssigphase Gelöste wird durch Partialdruckerniedrigung abgetrennt; Desorption und Strippung werden häufig einander überlagernd angewendet) beruht die Trennwirkung insbesondere auf der unterschiedlichen Löslichkeit von (Meth)acrylmonomeren und von diesen verschiedenen Nebenkomponenten in einer Flüssigkeit. Bei der Destillation und Rektifikation beruht die resultieren Trennwirkung vor allem auf der Verschiedenheit der Siedepunkte von (Meth)acrylmonomeren und Nebenkomponenten. Die azeotrope Destillation bzw. Rektifikation nützen die unterschiedlich ausgeprägte Neigung von (Meth)acrylmonomeren und Nebenkomponenten zur Ausbildung von Azeotropen mit zugesetzten azeotropen Schleppern aus.

Das erfindungsgemäße Verfahren ist insbesondere dann von Bedeutung, wenn bei einem thermischen Trennverfahren ein flüssiger, wenigstens ein (trennwirksam zu behandelnder) (Meth)acrylmonomeres (z. B. Acrylsäure oder Methacrylsäure) enthaltender Stoffstrom zu Trennzwecken in eine trennwirksame Einbauten enthaltende Trennkolonne geführt wird, und das erfindungsgemäß zu erwärmende, wenigstens ein (Meth)acrylmonomeres enthaltende flüssige Gemisch dem die Trennkolonne umfassenden trennwirksamen Raum unterhalb der Zufuhrstelle des trennwirksam zu behandelnden (flüssigen) Stoffstroms in die Trennkolonne entnommen, mit Hilfe eines indirekten Wärmeaustauschers erfindungsgemäß erwärmt und so erwärmt unterhalb der Zufuhrstelle des trennwirksam zu behandelnden (flüssigen) Stoffstroms in die Trennkolonne, in den die Trennkolonne umfassenden trennwirksamen Raum rückgeführt wird.

Das Vorgenannte gilt insbesondere dann, wenn Acrylsäure oder Methacrylsäure das (Meth)acrylmonomere ist und der trennwirksam zu behandelnde flüssige Stoffstrom im wesentlichen diejenige flüssige Phase ist, die dann entsteht, wenn man die Acrylsäure oder Methacrylsäure aus dem Produktgasgemisch einer heterogen katalysierten partiellen Gasphasenoxidation zur Herstellung von Acrylsäure oder Methacrylsäure aus einer C₃- bzw. C₄-Vorläuferverbindung durch absorptive und/oder kondensative Maßnahmen aus dem Produktgasgemisch in die flüssige Phase überführt (vgl. z. B. DE-A 103 363 86, WO 01/96271, DE-A 196 316 45, DE-A 195 013 25, EP-A 982 289, DE-A 198 388 45, WO 02/076917, EP-A 1695 954, EP-A 695 736, EP-A 778 225, EP-A 1041 062, EP-A 982 287, EP-A 982 288, US 2004/024 2826, EP-A 792 867, EP-A 784 046, EP-A 695 736, EP-A 112 5912 und die in diesen Schriften diesbezüglich zitierte Literatur, sowie die deutsche Anmeldung 102006049939.5).

Dies trifft insbesondere dann zu, wenn die Trennkolonne bei vermindertem Druck (z. B. einem Kopfdruck von 20 bis 100, bzw. bis 150 mbar) betrieben wird und die Rückführtemperatur aus dem indirekten Wärmeaustauscher heraus in den trennwirksamen Raum ≥ 150 °C, oder ≥ 180 °C, oder ≥ 200 °C, oder 220 °C oder mehr beträgt.

Grundsätzlich kann die erfindungsgemäß zuzusetzende, Stickstoff enthaltende, Wirkverbindung erst kurz vor dem Eintritt des erfindungsgemäß zu erwärmenden flüssigen Gemischs in den indirekten Wärmeaustauscher und/oder bereits in den trennwirksamen Raum hinein zudosiert werden, dem das erfindungsgemäß zu erwärmende flüssige Gemisch entnommen wird. Da normalerweise aus dem trennwirksamen Raum auch schwersiedende Fraktion ausgeschleust wird, muss die Wirkverbindung in der Regel stetig nachdosiert werden.

Häufig werden als Trennkolonnen mit trennwirksamen Einbauten für thermische Trennverfahren solche verwendet, die wenigstens als einen Teil der trennwirksamen Einbauten eine Abfolge von Stoffaustauschböden enthalten. Stoffaustauschböden verfolgen den Zweck, in der Trennkolonne in Form von Flüssigkeitsschichten Orte mit geschlossenen flüssigen Phasen zur Verfügung zu stellen. Die Oberfläche des in der Flüssigkeitsschicht z. B. aufsteigenden und sich dabei in der geschlossenen flüssigen Phase verteilenden Dampf- bzw. Gasstromes ist dann die maßgebende Austauschfläche. Stoffaustauschböden schließen mit der sie umgebenden Wandung vorzugsweise dicht ab.

Ein Klassiker unter den Stoffaustauschböden ist der Siebboden. Darunter werden in dieser Schrift Platten verstanden, die als Durchtrittsstellen für die aufsteigende Gas- bzw. Dampfphase einfache Löcher und/oder Schlitze aufweisen.

Die Siebböden werden dabei üblicherweise in zwei Gruppen differenziert, nämlich in solche mit Flüssigkeitszwangsführung und solche ohne Flüssigkeitszwangsführung.

Ganz generell wird Flüssigkeitszwangsführung bei Stoffaustauschböden dadurch erzielt, dass die Stoffaustauschböden wenigstens einen Ablaufschacht aufweisen (Ablauf), durch den die Flüssigkeit unabhängig vom Strömungsweg des Dampfes vom höher gelegenen Boden auf den tiefer gelegenen Boden fließt (Zulauf). Die horizontale Flüssigkeitsströmung über den Austauschboden vom Zulauf zum Ablauf wird entsprechend der verfahrenstechnischen Aufgabenstellung gewählt. Das Gas bzw. der Dampf tritt durch die offenen Querschnitte der Bodenplatte.

Wird die Flüssigkeit im Umkehrstrom über den Boden geführt (Zulauf und Ablauf des Stoffaustauschbodens sind auf der gleichen Seite des Bodens angeordnet), spricht man von Umkehrstromböden. Bei Radialstromböden strömt die Flüssigkeit auf dem Boden radial von der Mitte (Zulauf) zum Ablauf am Rand des Bodens.

Bei den Querstromböden wird die Flüssigkeit, über den gesamten Fließbereich betrachtet, quer über den Boden vom Zulauf zum Ablauf geführt. In der Regel sind Querstromböden einflutig gestaltet. D.h., Zulauf und Ablauf sind auf gegenüberliegenden Seiten des Bodens angeordnet. Sie können aber auch zweiflutig (oder auch mehr als zweiflutig) gestaltet sein. In diesem Fall kann der Zulauf z. B. in der Mitte und je ein Ablauf auf gegenüberliegenden Seiten des Stoffaustauschbodens angeordnet sein.

D. h., bei Siebböden wird die Flüssigkeitszwangsführung dadurch erzielt, dass die Siebböden neben den Durchtrittsstellen für die aufsteigende Gas- bzw. Dampfphase wenigstens einen Ablaufschacht aufweisen (Ablauf), durch den die Flüssigkeit unabhängig vom Strömungsweg des Dampfes vom höher gelegenen Boden auf den nächsten tiefer gelegenen Boden fließt (Zulauf). Die Flüssigkeit fließt z. B. im Querstrom über den Boden vom wenigstens einen Zulauf zum wenigstens einen Ablauf, wobei das Zulauf- und Ablaufrohr den Flüssigkeitsverschluss und die gewünschte Flüssigkeitshöhe auf dem Boden garantieren. Häufig (insbesondere bei geringen Kolonnendurchmessern) sind die Siebböden mit Flüssigkeitszwangsführung einflutig gestaltet. D.h., Zulauf und Ablauf sind auf gegenüberliegenden Seiten des Bodens angeordnet. Sie können aber auch zweiflutig (oder auch mehr als zweiflutig) gestaltet sein. In diesem Fall kann der Zulauf z. B. in der Mitte und je ein Ablauf auf gegenüberliegenden Seiten des Stoffaustauschbodens angeordnet sein. Nachfolgend sollen solche Siebböden als Zwangssiebböden bezeichnet werden. Bei ihnen wird ein die Trennwirkung minderndes Durchregnen der Flüssigkeit nicht wie beim hydraulisch abgedichteten Querstromboden durch Kamine verhindert, in die die Durchtrittsöffnungen fortführen, sondern es bedarf dazu einer minimalen Dampfbelastung. Der Dampf tritt aufsteigend durch die Durchtrittsöffnungen und durchperlt die vom Ablaufrohr gehaltene Flüssigkeitsschicht.

Von den Zwangssiebböden unterscheiden sich die Dual-Flow- oder auch Regensiebböden dadurch, dass sie kein Ablaufsegment enthalten. Durch die Abwesenheit von Ablaufsegmenten (Ablaufschächten) treten bei den Regensiebböden das aufsteigende Gas und die in der Trennkolonne absteigende Flüssigkeit durch die gleichen Durchtrittsstellen des Bodens. Auch beim Regensiebboden bedarf es wie beim Zwangssiebboden einer minimalen Dampfbelastung, um eine angemessene Trennwirkung zu erzielen. Wird sie signifikant unterschritten, bewegen sich aufsteigendes Gas und absteigender Rücklauf im wesentlichen ohne Austausch aneinander vorbei und der Boden läuft Gefahr, trocken zu laufen.

D. h., auch beim Regensiebboden muss eine untere Grenzgeschwindigkeit vorhanden sein, damit auf dem Boden eine gewisse Flüssigkeitsschicht gehalten wird, um ein Arbeiten des Bodens zu ermöglichen. Im normalen Arbeitsbereich regnet die Flüssigkeit bei Regensiebböden durch die Durchtrittsöffnungen von Boden zu Boden und zwischen den Böden wird die geschlossene Gasphase von einer zerteilten Flüssigkeitsphase durchsetzt.

Hydraulisch abgedichtete Querstromböden sind gegenüber Siebböden dadurch charakterisiert, dass sie beim Abschalten der Kolonne nicht leer laufen können, sieht man von der winzigen Leerlaufbohrung (ihr Querschnitt ist normalerweise mehr als 200 mal kleiner als der Gesamtquerschnitt der Durchtrittsstellen) ab, die jeder Querstromboden aus Zweckmäßigkeitsgründen aufweist.

D. h., auch bei geringen Kolonnenbelastungen weisen hydraulisch abgedichtete Querstromböden gestaute Flüssigkeit (Rücklauf- und/oder Zulaufflüssigkeit) auf und laufen keine Gefahr, trocken zu laufen. Dies ist dadurch bedingt, dass es sich bei den Durchtrittstellen von hydraulisch abgedichteten Querstromböden nicht wie bei Siebböden um kaminlose Bohrungen handelt. Vielmehr mündet jede Durchtrittstelle in einen Kamin, der ein Trockenlaufen unterbindet. Über dem Kamin sind Dampfumlenkhauben (Glocken) angebracht, die in die gestaute Bodenflüssigkeit eintauchen. Häufig sind die Dampfumlenkhauben an ihren Rändern geschlitzt oder gezackt (d. h., sie weisen Treibschlitze auf). Der durch die Durchtrittsstelle aufsteigende Dampfstrom erfährt durch die Dampfumlenkhauben eine Ablenkung und strömt parallel zum Boden, d.h., quer zur Kolonne, in die gestaute Flüssigkeit.

Die aus benachbarten, in der Regel über dem Boden äquidistant verteilt angeordneten, Hauben austretenden Dampfblasen bilden in der gestauten Flüssigkeit eine Sprudelschicht aus.

Ablaufrohre bzw. -segmente, die, in der Regel abwechselnd links oder rechts, Böden verlassen, regeln - von Wehren unterstützt - den Flüssigkeitsstand der Stoffaustauschböden und führen die Flüssigkeit dem darunter liegenden Boden zu. Für die hydraulisch abdichtende Wirkung ist wesentlich, dass die Ablaufrohre bzw. -Segmente des oberen Bodens in die gestaute Flüssigkeit des darunter liegenden Bodens tauchen. Vorzugsweise sind keine Zulaufwehre vorhanden. In der Höhe einstellbare Glocken gestatten ein Anpassen an die Strömungsverhältnisse und den Ausgleich der Eintauchtiefen bei Herstellungsungleichmäßigkeiten, so dass alle Glocken des Bodens gleichmäßig gasen.

Je nach Gestalt und Anordnung der Glocken unterscheidet man z. B. die einflutig gestalteten hydraulisch abgedichteten Querstromböden in Rundglockenböden (Durchtrittstelle, Kamin und Glocke sind rund), Tunnel-Böden (Durchtrittstelle, Kamin und Glocke sind rechteckig, die Glocken sind hintereinander angeordnet, wobei die längere Rechteckkante parallel zur Querstromrichtung der Flüssigkeit ausgerichtet ist) und Thormann-Böden (Durchtrittstelle, Kamin und Glocke sind rechteckig, die Glocken sind hintereinander angeordnet, wobei die längere Rechteckkante senkrecht zur Querstromrichtung der Flüssigkeit ausgerichtet ist).

Unter Ventilböden sollen in dieser Schrift Querstromböden verstanden werden, die Bodenbohrungen mit hubbegrenzten Teller-, Ballast - oder Hebeventilen (Schwimmklappen) aufweisen, die die Größe der Dampfdurchtrittsöffnung der jeweiligen Kolonnenbelastung anpassen. Der aufsteigende Gasstrom wird abgelenkt, strömt parallel zum Boden in die gestaute Rücklaufflüssigkeit und bildet eine Sprudelschicht aus. Bewehrte Ablaufrohre führen den Rücklauf von Boden zu Boden. Häufig sind sie zweiflutig gestaltet. Sie können aber auch drei - und mehrflutig (z. B. bis zu achtflutig) gestaltet sein.

Stoffaustauschböden, auf denen Gleichgewicht herrscht zwischen ablaufender Flüssigkeit und aufsteigendem Dampf werden als theoretische Böden bezeichnet.

Dieser Begriff lässt sich sowohl auf alle anderen für Gegenstromdestillationen (Rektifikationen) geeigneten trennwirksamen Einbauten übertragen (wie Packungen und Füllkörperschüttungen) als auch auf andere thermische Trennvorgänge wie die Desorption und Strippung.

Es ist deshalb zweckmäßig, allgemein von theoretischen Trennstufen zu sprechen. Als theoretische Trennstufe wird die Raumeinheit definiert, die eine Anreicherung entsprechend dem thermodynamischen Gleichgewicht bewirkt.

Häufig erfolgt die Überführung von Acrylsäure aus dem Produktgasgemisch einer heterogen katalysierten Gasphasen-Partialoxidation wenigstens einer C₃-Vorläuferverbindung der Acrylsäure (z. B. Propylen, Acrolein und/oder Propan) bei erhöhter Temperatur mit molekularem Sauerstoff an im festen Aggregatzustand befindlichen Katalysatoren in die flüssige Phase z.B. dadurch, dass man das heiße, die Acrylsäure enthaltende Produktgasgemisch, gegebenenfalls nach indirekter und/oder direkter Abkühlung desselben, in eine mit trennwirksamen Einbauten (vorzugsweise Stoffaustauschböden) ausgerüstete Kondensationskolonne leitet und innerhalb der Kondensationskolonne in sich selbst aufsteigen lässt, dabei fraktionierend kondensiert und im Seitenabzug eine rohe Acrylsäure aus der Kondensationskolonne entnimmt, deren Acrylsäuregehalt in der Regel ≥ 90 Gew.-%, vielfach sogar ≥ 95 Gew.-% beträgt (vgl. z. B. DE-A 102 358 47, WO 2000/53560, DE-A 102 436 25, WO 2004/035514 und DE-A 103 327 58). Die für diese Auftrennung des Produktgasgemischs der Gasphasen-Partialoxidation erforderliche thermische Energie bringt das heiße Produktgasgemisch im wesentlichen bereits mit.

Als Auslass für die schwerer als Acrylsäure siedenden Nebenkomponenten wird aus dem Sumpf der Kondensationskolonne diese Nebenkomponenten enthaltende Sumpfflüssigkeit oder über einen unterhalb des Seitenabzugs für die rohe Acrylsäure gelegenen Seitenabzug diese Nebenkomponente enthaltende Schwersiederfraktion oder ein Gemisch aus solcher Sumpfflüssigkeit und Schwersiederfraktion entnommen (nachfolgend alle übergeordnet als Schwersiederflüssigkeit bezeichnet. Eine Teilmenge der vorgenannten Schwersiederflüssigkeit kann zur Direktkühlung des Produktgasgemischs der Gasphasen-Partialoxidation verwendet und über diese Direktkühlung im Schwersiederbereich der Kondensationskolonne in diese rückgeführt werden. Die nicht auf diesem Weg in die Kondensationskolonne rückgeführte, der Kondensationskolonne entnommene, Schwersiederflüssigkeit enthält noch vergleichsweise große Mengen an Acrylsäure. Um zu vermeiden, dass diese Acrylsäure gemeinsam mit den schwersiedenden Nebenkomponenten der Entsorgung zugeführt wird (d.h., um die Ausbeute an Acrylsäure zu erhöhen), unterwirft man die Schwersiederflüssigkeit vorab dieser Entsorgung daher vorteilhaft bei erhöhter Temperatur einer Strippung. Als Strippgas wird zweckmäßigerweise von dem die Kondensationskolonne an deren Kopf verlassendem, und vor allem die schwerstkondensierbaren Bestandteile des Produktgasgemischs der Gasphasen-Partialoxidation enthaltenden, Restgas verwendet. Dazu wird von selbigem zweckmäßig eine entsprechende Teilmenge komprimiert und überhitzt (in der Regel auf die im Sumpf der Strippkolonne herrschende Temperatur). Die Strippung selbst wird anwendungstechnisch vorteilhaft in einer trennwirksame Einbauten (vorzugsweise äquidistante Regensiebböden) enthaltenden Rektifikationskolonne (Strippkolonne) durchgeführt, in deren unteren Teil (unteres Drittel der theoretischen Böden) die zu strippende Schwersiederflüssigkeit mit Vorteil zugeführt wird.

Um eine möglichst hohe Strippeffizienz zu gewährleisten, wird in zielgerichteter Weise aus dem Sumpf der Strippkolonne kontinuierlich ein Acrylsäure enthaltendes flüssiges Gemisch entnommen, zum Zweck seiner Erwärmung durch einen indirekten Wärmeaustauscher I geführt (in der Regel ein Zwangsumlaufwärmeaustauscher (häufig ein Zwangsumlaufrohrbündelwärmeübertrager), häufig ein Zwangsumlaufentspannungswärmeaustauscher (vielfach ein Zwangsumlaufrohrbündelentspannungswärmeübertrager)) und anschließend zum überwiegenden Teil erhitzt in die Rektifikationskolonne zurückgefördert (zweckmäßigerweise in den Sumpf). Das Strippgas wird vorzugsweise ebenfalls dem Sumpf der Strippkolonne zugeführt. Der andere Teil der im Wärmeaustauscher I erhitzten Sumpfflüssigkeit aus der Strippkolonne wird Viskositäts-(bevorzugt), Dichte- oder Temperatur-geregelt in einen Behälter gefahren, in selbigem entgast und mit Methanol verdünnt der Rückstandsverbrennung zugeführt.

In der Strippkolonne steigt ein Acrylsäure enthaltendes Gasgemisch in selbiger auf. Im Gegenstrom wird oberhalb der Zufuhrstelle der Schwersiederflüssigkeit vorteilhaft Rücklaufflüssigkeit geführt, um eine erhöhte Trennwirkung insbesondere gegenüber den schwersiedenden Nebenkomponenten zu gewährleisten, dem Siedepunkt von demjenigen der Acrylsäure nicht sehr verschieden ist. Zur Erzeugung der Rücklaufflüssigkeit wird das z.B. durch einen Kaminboden, der die trennwirksamen Einbauten im oberen Bereich der Strippkolonne abschließt, geführte Gasgemisch hinter demselben durch direkte Kühlung in einem Sprühkühler abgekühlt und partiell kondensiert. Das überwiegend aus Acrylsäure bestehende Kondensat wird vom Kaminboden, der gleichzeitig als Fangboden fungiert, aufgefangen und von selbigem entnommen. Eine Teilmenge wird in einem indirekten Wärmeaustauscher II (vorzugsweise ein Plattenwärmeaustauscher) abgekühlt (z. B. mittels Wasser als Wärmeträger) und nachfolgend als Kühlflüssigkeit für die direkte Sprühkühlung wieder verwendet (rückgeführt). Zum Zweck der Polymerisationsinhibierung wird der diesbezüglich entnommenen Kondensat-Teil- oder Gesamtmenge vor ihrer Abkühlung im indirekten Wärmeaustauscher II vorteilhaft eine weitere Menge an zu strippender (Polymerisationsinhibitoren enthaltender) Schwersiederflüssigkeit (aus der Kondensationskolonne entstammender) zugeführt und eine Teilmenge des dabei resultierenden Gemischs vor dem Eintritt desselben in den Wärmeaustauscher II im wesentlichen unmittelbar unterhalb des Kaminbodens als Rücklaufflüssigkeit in die Strippkolonne rückgeführt. Im Bedarfsfall kann eine Teilmenge an vom Kaminboden entnommenem Kondensat auch direkt in den Sumpf der Kondensationskolonne rückgeführt werden.

Der aus der Strippkolonne gasförmig entweichende, im Rahmen der Sprühkühlung nicht kondensierte Gasstrom, der die freigestrippte Acrylsäure trägt, wird anwendungstechnisch zweckmäßig mit dem aus der Gasphasen-Partialoxidation kommenden Produktgasgemisch vereinigt (vorzugsweise z.B. im Rahmen der Direktkühlung desselben oder im Sumpfraum der Kondensationskolonne (vorzugsweise nicht getaucht in selbigen rückgeführt). Die die Kondensationskolonne verlassende, nicht zum Strippen verwendete, Restgasmenge wird bei Bedarf teilweise als inertes Verdünnungsgas in die heterogen katalysierte Gasphasen-Partialoxidation rückgeführt und die dort nicht verwendbare Restmenge wird entsorgt, z. B. verbrannt.

Der Arbeitsdruck innerhalb der Strippkolonne liegt regelmäßig oberhalb von Atmosphärendruck. Oberhalb der trennwirksamen Einbauten in der Strippkolonne sind Arbeitsdrucke von 1,3 bis 2 bar typisch. Die Erzeugung der Rücklaufflüssigkeit für die Strippkolonne kann grundsätzlich auch aus der Strippkolonne ausgelagert erfolgen. Die Flüssigkeit im Sumpf der Strippkolonne befindet sich vorzugsweise im Siedezustand. Ist der Wärmeaustauscher I als ein Zwangsumlaufrohrbündelentspannungswärme-überträger ausgebildet, so ist dieser im Unterschied zum Fall eines reinen Zwangsumlaufrohrbündelwärmeübertragers von der Strippkolonne normalerweise durch eine Drosselvorrichtung (z. B. im einfachsten Fall durch eine Lochblende; alternativ kommt auch ein Ventil in Betracht) getrennt. Der bei einem Phasengrenzdruck Pₓ befindlichen, Acrylsäure enthaltenden, vorzugsweise siedenden Sumpfflüssigkeit aus der Strippkolonne wird kontinuierlich ein Teil entnommen und mittels einer Umlaufpumpe in die Zuströme eines Rohrbündelwärmeaustauschers gepumpt. Um die innenliegenden Rohre des Rohrbündelwärmeübertragers strömt ein fluider Wärmeträger (z.B. Heizdampf; d. h., unter Druck stehender Wasserdampf), dessen Temperatur oberhalb der Temperatur der Sumpfflüssigkeit in der Strippkolonne liegt. Auf dem Weg durch die Zu-und Ausströmrohre des Rohrbündelwärmeaustauschers wird die der Strippkolonne entnommene Sumpfflüssigkeit durch indirekten Wärmeaustausch auf eine Temperatur T_{y'} erhitzt, die oberhalb der Temperatur des Sumpfes der Strippkolonne liegt. Die bereits erwähnte Drosselvorrichtung trennt den Rohrbündelwärmeübertrager und die Strippkolonne druckseitig und ermöglich durch geeignete Wahl der Umlaufpumpenleistung die Einstellung eines oberhalb von Pₓ gelegenen Drosselvordrucks P_{y}, der oberhalb des zur Temperatur T_{y'} gehörigen Siededrucks P_{y'} der entnommenen Strippkolonnensumpfflüssigkeit liegt. Durch vorstehende Maßnahmen wird ein Sieden des umgepumpten Strippkolonnensumpfflüssigkeitsanteils in den Rohren des Rohrbündelwärmeübertragers unterdrückt.

Der umgepumpte Anteil der Strippkolonnenflüssigkeit wird in den Rohren des Rohrbündelwärmeübertragers bezüglich des über dem Flüssigkeitsspiegel der Sumpfflüssigkeit in der Strippkolonne herrschenden Druckes Pₓ vielmehr überhitzt und der Siedeprozess so auf die Durchtrittseite der Drosselvorrichtung verlagert (d. h., der Inhalt der Rohre des Rohrbündelwärmeübertragers liegt einphasig vor, der Rohrbündelwärmeübertrager fungiert lediglich als Überhitzer). Der Durchtritt der so überhitzten "Sumpfflüssigkeit" durch die Drosselvorrichtung in die Strippkolonne kann dabei unmittelbar oder mittelbar in die Strippkolonnenflüssigkeit hinein erfolgen. Unter diesen Bedingungen entspricht die Temperatur des flüssigen Strippkolonnensumpfes regelmäßig der zum (unmittelbar) über der Sumpfflüssigkeit herrschenden Druck Pₓ gehörigen Siedetemperatur Tₓ. Prinzipiell kann der Durchtritt der wie beschrieben überhitzten Sumpfflüssigkeit durch die Drosselvorrichtung in die Strippkolonne aber auch oberhalb des Flüssigkeitsspiegels des Strippkolonnensumpfes und nicht in diesen hineingerichtet erfolgen. Unter diesen Bedingungen liegt die Temperatur des flüssigen Strippkolonnensumpfes regelmäßig unterhalb der zum (unmittelbar) über der Sumpfflüssigkeit herrschenden Druck Pₓ gehörigen Siedetemperatur Tₓ. Wesentlich ist, dass die Siedeverdampfung des außerhalb der Strippkolonne angebrachten Rohrbündelwärmeübertragers erst in der Strippkolonne, d.h., außerhalb des Zwangsumlaufrohrbündelwärmeübertragers, eintritt. Die Drosselung kann, wie bereits erwähnt, z. B. mechanisch (Blenden, Ventile) und/oder hydrostatisch (durch eine entsprechend hohe Sumpfsäule über der Durchtrittstelle der überhitzten "Sumpfflüssigkeit") erfolgen. Die Temperatur im Sumpf der Strippkolonne wird in typischer Weise 150 bis 180°C, häufig 160 bis 170°C betragen. Die Temperatur beim Verlassen des Zwangsumlaufrohrbündelwärmeüberträgers liegt in der Regel wenigstens 5°C oberhalb der Sumpfentnahmetemperatur. Der Kaminboden in der Strippkolonne ist mit Vorteil ein solcher, der die vorteilhaften Eigenschaften gemäß der DE-A 102 005 009 469 sowie der DE-A 101 598 825 in sich vereint. Die den Zwangsumlauf bewältigende Pumpe ist mit Vorteil eine solche mit doppelt wirkender Gleitringdichtung gemäß der DE-A 102 288 59, wobei als Sperrflüssigkeit anwendungstechnisch zweckmäßig ein Glykol/Wasser-Gemisch verwendet wird.

Wesentlich im Fall der vorbeschriebenen Verfahrensweise ist, dass der indirekte Wärmeaustauscher I, mit Hilfe dessen die Acrylsäure enthaltende, der Strippkolonne entnommene, Sumpfflüssigkeit erwärmt und dadurch auf dem Weg der Rückführung von solchermaßen erwärmter Sumpfflüssigkeit in die Strippkolonne die zur Durchführung des thermischen Trennverfahrens "Strippen" benötigte thermische Energie zur Verfügung gestellt wird, möglichst lange ungestört, d.h., insbesondere frei von Fouling der Rohrbündelwärmeübertragerrohre, betrieben werden kann. Zu diesem Zweck wird man erfindungsgemäß vorteilhaft unmittelbar in den Sumpf der Strippkolonne eine erfindungsgemäß empfohlene Wirkverbindung zusetzen. Für das vorstehend beschriebene spezielle indirekte Wärmeübertragungsproblem eignen sich mit besonderem Vorteil als solche Wirkverbindungen das Triethylamin, das N,N,N',N'-Tetramethyl-1,3-propan-diamin und das Pentamethyldiethylentriamin. Aber auch alle anderen in dieser Schrift empfohlenen Wirkverbindungen sind grundsätzlich an dieser Einsatzstelle geeignet. Die Zufuhr der Wirkverbindung in die Sumpfflüssigkeit der Strippkolonne kann dabei in Substanz befindlich oder z. B. auch in der Kondensationskolonne über Seitenabzug entnommener roher Acrylsäure gelöst erfolgen. Erfindungsgemäß bevorzugt sind dabei solche Lösungen, die an der Wirkverbindung möglichst konzentriert sind. Eine typische Einsatzmenge an erfindungsgemäßer Wirkverbindung beim vorbeschriebenen indirekten Wärmeübertragungsproblem liegt bei 0,5 bis 1 Gew.-% (aber auch 0,1 bis 10 Gew.-% sind möglich), bezogen auf die in den Zwangsumlaufrohrbündelentspannungswärmeübertrager geführte Menge an Sumpfflüssigkeit.

Ein Vorteil der erfindungsgemäßen Wirkverbindungen liegt auch darin begründet, dass es sich entweder bereits um ionische Verbindungen handelt, oder um solche Verbindungen, die mit Acrylsäure und/oder Methacrylsäure ionische Verbindungen auszubilden vermögen. Der Vorteil ionischer Verbindungen liegt aber unter anderem darin begründet, dass sie einen erhöhten Siedepunkt aufweisen und deshalb insbesondere thermische Trennverfahren besonders störungsfrei zu begleiten vermögen.

Selbstredend können die erfindungsgemäß zuzusetzenden Wirkverbindungen auch gemeinsam mit den anderen im Stand der Technik bereits empfohlenen Antifoulingmitteln (insbesondere einem Tensid, z. B. einem solchen gemäß EP-A 1062 197) den erfindungsgemäß zu erwärmenden, wenigstens ein (Meth)acrylmonomeres enthaltenden, flüssigen Gemischen zugesetzt werden. Als solche kommen u.a. auch diejenigen der US-A 3,271,296 und des GB-Patents Nr. 922 831 in Betracht.

Abschließend sei vermerkt, dass nach den Verfahren gemäß den Schriften WO 2004/035514, DE-A 103 327 58, DE-A 102 436 25, WO 2000/53560 und DE-A 102 358 47 durch Fehloperationen gegebenenfalls nicht spezifikationsgerecht produzierte reine Acrylsäure in einfacher Weise dadurch wieder aufgearbeitet werden kann, dass man sie gemeinsam mit aus der Kondensationskolonne entnommener zu strippender Schwersiederflüssigkeit der vorstehend beschriebenen Strippkolonne zuführt. Die auf diese Weise in die Strippkolonne zusätzlich zugeführte Acrylsäure wird in der gleichen Weise wie die in der Schwersiederflüssigkeit enthaltene Acrylsäure herausgestrippt und als Bestandteil des aus der Strippkolonne entnommenen mit Acrylsäure beladenen Strippgases (bzw. gegebenenfalls) in den Sumpf der Kondensationskolonne (bzw. in die Direktkühlung des Produktgasgemischs der Partialoxidation) und damit in den Prozess zur Gewinnung von reiner Acrylsäure rückgeführt.

Damit umfasst vorliegende Erfindung insbesondere die folgenden erfindungsgemäßen Ausführungsformen:
1. Ein Verfahren zur Übertragung von Wärme auf ein wenigstens ein (Meth)acryl-monomeres enthaltendes flüssiges Gemisch mit Hilfe eines indirekten Wärmeaustauschers, der auf seiner Primärseite von einem fluiden Wärmeträger und auf seiner Sekundärseite gleichzeitig von dem wenigstens ein (Meth)acrylmono-meres enthaltenden flüssigen Gemisch durchströmt wird, dadurch gekennzeichnet, dass das wenigstens ein (Meth)acrylmonomeres enthaltende flüssige Gemisch wenigstens eine von (Meth)acrylmonomeren verschiedene Wirkverbindung aus der Gruppe bestehend aus tertiären Aminen, den Salzen gebildet aus einem tertiären Amin und einer Brönsted-Säure, sowie quartären Ammoniumverbindungen mit der Maßgabe zugesetzt enthält, dass von den tertiären und quartären Stickstoffatomen in der wenigstens einen Wirkverbindung keines eine Phenylgruppe, aber wenigstens eine Teilmenge wenigstens eine Alkylgruppe trägt.
2. Ein Verfahren gemäß Ausführungsform 1, dadurch gekennzeichnet, dass das wenigstens eine (Meth)acrylmonomere Acrylsäure und/oder Methacrylsäure ist.
3. Ein Verfahren gemäß Ausführungsform 1, dadurch gekennzeichnet, dass das wenigstens eine (Meth)acrylmonomere wenigstens ein Monomeres aus der Gruppe bestehend aus Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, iso-Butylmethacrylat, n-Butylmethacrylat, tert.-Butylacrylat, tert.-Butylmethacrylat, Ethylacrylat, Ethylmethacrylat, 2-Ethylhexylacrylat und 2-Ethylhexylmethacrylat ist.
4. Ein Verfahren gemäß einer der Ausführungsformen 1 bis 3, dadurch gekennzeichnet, dass die wenigstens eine Wirkverbindung ein tertiäres Amin der allgemeinen Formel 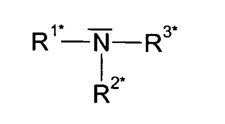 mit
   - R¹*, R²* und R³*: unabhängig voneinander eine 1 bis 8 C-Atome aufweisende Al- kylgruppe, oder eine 1 bis 8 C-Atome aufweisende Alkylgruppe, bei der ein oder mehrere Wasserstoffatome durch wenigstens eine der Gruppen -OH, -NH₂, -NHCH₃ und -N(CH₃)₂ ersetzt und/oder die Kohlenstoffkette wenigstens einmal durch ein Sauerstoffatom unterbrochen ist, oder eine 1 bis 8 C-Atome aufweisende Cycloalkylgruppe bei der ein oder mehr Wasser- stoffatome durch wenigstens eine der Gruppen -OH, -NH₂, -NHCH₃ und -N(CH₃)₂ ersetzt und/oder die cyclische Kohlen- stoffkette wenigstens einmal durch ein Sauerstoffatom unter- brochen ist,
   oder das Salz aus einem solchen tertiären Amin und einer Brönsted-Säure ist.
5. Ein Verfahren gemäß einer der Ausführungsformen 1 bis 3, dadurch gekennzeichnet, dass die wenigstens eine Wirkverbindung ein Derivat des 1,3-Diazols ist, das sich von diesem dadurch ableitet, dass der Wasserstoff am Stickstoff in der 1-Stellung durch eine Alkylgruppe R⁴ mit 1 bis 8 C-Atomen ersetzt und/oder der Stickstoff des 1,3-Diazols in der 3-Stellung mit einer Alkylgruppe R⁵ mit 1 bis 8 C-Atomen alkyliert ist.
6. Ein Verfahren gemäß einer der Ausführungsformen 1 bis 3, dadurch gekennzeichnet, dass die wenigstens eine Wirkverbindung das Salz eines quartären Ammoniumions ist, wobei das quartäre Ammoniumion ein solches der allgemeinen Formel 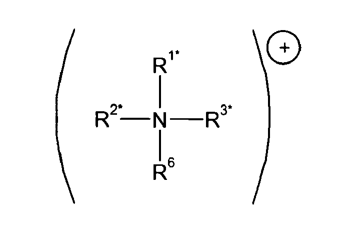 mit
   - R¹*, R²* und R³*: unabhängig voneinander und unabhängig von R⁶ eine 1 bis 8 C-Atome aufweisende Alkylgruppe, oder eine 1 bis 8 C-Atome aufweisende Alkylgruppe, bei der ein oder mehrere Wasser- stoffatome durch wenigstens eine der Gruppen -OH, -NH₂, -NHCH₃ und -N(CH₃)₂ ersetzt und/oder die Kohlenstoffkette wenigstens einmal durch ein Sauerstoffatom unterbrochen ist, oder eine 1 bis 8 C-Atome aufweisende Cycloalkylgruppe, oder eine 1 bis 8 C-Atome aufweisende Cycloalkylgruppe, bei der ein oder mehr Wasserstoffatome durch wenigstens eine der Gruppen -OH, -NH₂, -NHCH₃ und -N(CH₃)₂ ersetzt und/oder
   - R⁶: die cyclische Kohlenstoffkette wenigstens einmal durch ein Sauerstoffatom unterbrochen ist, und unabhängig von R¹*, R²* und R³* eine 1 bis 8 C-Atome aufwei- sende Alkylgruppe,
   ist.
7. Ein Verfahren gemäß einer der Ausführungsformen 1 bis 6, dadurch gekennzeichnet, dass das Molgewicht der wenigstens einen Wirkverbindung ≤ 600 g beträgt.
8. Ein Verfahren gemäß einer der Ausführungsformen 1 bis 7, dadurch gekennzeichnet, dass das flüssige Gemisch 0,01 bis 10 Gew.-% seines Gewichtes von der wenigstens einen Wirkverbindung zugesetzt enthält.
9. Ein Verfahren gemäß einer der Ausführungsformen 1 bis 8, dadurch gekennzeichnet, dass das flüssige Gemisch zur ≥ 0,5 Gew.-% seines Gewichtes von dem wenigstens einen (Meth)acrylmonomeren enthält.
10. Ein Verfahren gemäß einer der Ausführungsformen 1 bis 9, dadurch gekennzeichnet, dass die Temperatur, mit der das flüssige Gemisch den indirekten Wärmeaustauscher verlässt, 50 bis 350 °C beträgt.
11. Ein Verfahren gemäß einer der Ausführungsformen 1 bis 10, dadurch gekennzeichnet, dass der indirekte Wärmeaustauscher ein Rohrbündelwärmeüberträger ist.
12. Ein Verfahren gemäß einer der Ausführungsformen 1 bis 11, dadurch gekennzeichnet, dass der fluide Wärmeträger Wasserdampf ist.
13. Ein Verfahren gemäß einer der Ausführungsformen 1 bis 12, dadurch gekennzeichnet, dass das flüssige Gemisch aus dem Sumpf einer trennwirksame Einbauten enthaltenden Trennkolonne entnommen wurde, in die im Rahmen eines thermischen Trennverfahrens wenigstens ein wenigstens ein (Meth)acrylmonomeres enthaltender Stoffstrom zugeführt und wenigstens ein von diesem verschiedener wenigstens ein (Meth)acrylmonomeres enthaltender Stoffstrom entnommen wird.
14. Ein Verfahren gemäß der Ausführungsform 13, dadurch gekennzeichnet, dass das flüssige Gemisch nach dem Verlassen des indirekten Wärmeaustauschers in die Trennkolonne rückgeführt wird.
15. Ein Verfahren gemäß der Ausführungsform 13 oder 14, dadurch gekennzeichnet, dass der wenigstens eine zugeführte Stoffstrom das Acrylsäure enthaltende Sumpfprodukt einer fraktionierenden Kondensation des Produktgasgemischs einer heterogen katalysierten Gasphasen-Partialoxidation einer C₃-Vorläuferverbindung zu Acrylsäure in einer Kondensationskolonne und das thermische Trennverfahren die Strippung der Acrylsäure aus diesem Sumpfprodukt ist.

### Beispiele und Vergleichsbeispiele

### 1. Vergleichsbeispiel

Aus einer zweistufigen heterogen katalysierten Gasphasen-Partialoxidation von Propylen zu Acrylsäure wurde ein Acrylsäure enthaltendes Produktgasgemisch entnommen und zur Gewinnung von reiner Acrylsäure wie in der DE-A 10332758 bzw. der WO 2004/035514 beschrieben einer fraktionierenden Kondensation unterworfen und die dabei über Seitenabzug entnommene rohe Acrylsäure gemäß der beiden vorgenannten Schriften weiterverarbeitet. Dem Sumpf der Kondensationskolonne wurden 2600 kg/h Sumpfflüssigkeit entnommen, die folgende Gehalte und eine Temperatur von 109,8 °C aufwies (nachfolgend als "Kondensationssumpfflüssigkeit" bezeichnet):

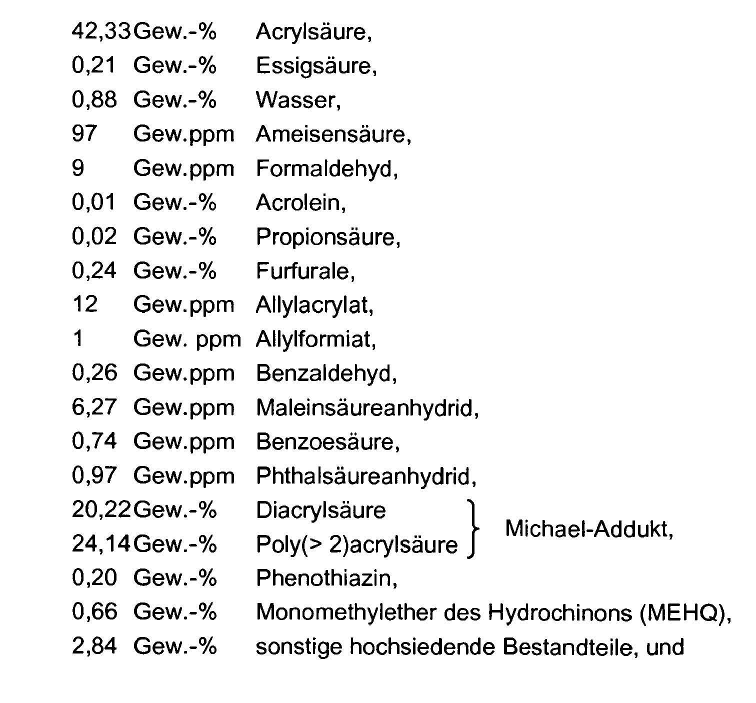

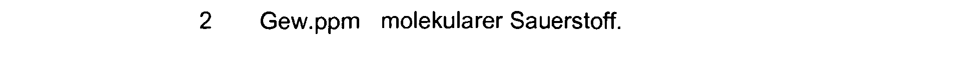

Eine erste Teilmenge von 1600 kg/h der vorgenannten entnommenen Sumpfflüssigkeit wurde in den unteren Teil einer Strippkolonne (Fertigungsmaterial = Edelstahl vom DIN-Typ 1.4571) zugeführt, die als trennwirksame Einbauten 50 Dual-Flow-Böden (Regensiebböden) enthielt. Der Innendurchmesser der Strippkolonne betrug über alle Dual-Flow-Böden einheitlich 2,4 m. Die Dual-Flow-Böden waren in der Strippkolonne äquidistant angeordnet, mit einem lichten Abstand von 400 mm. Ihr Öffnungsverhältnis betrug einheitlich 12 %. Die Lochdurchmesser (zur Anpassung an unterschiedliche Kolonnenbelastungen wird ein Teil der Lochöffnungen abgedeckt) der Dual-Flow-Böden betrug einheitlich 14 mm (Lochanordnung entsprechend strenger Dreiecksteilung; Abstand von Lochmitte zu Lochmitte = 26 mm (Böden 1 bis 4 von unten), 25,5, mm (Böden 5 bis 8 von unten) und 25 mm (Böden 9 bis 49 von unten) sowie 25,5 mm (Boden 50 von unten)). Die Bodendicke betrug jeweils 4 mm. Der unterste der Dual-Flow-Böden war 7435 mm oberhalb des unteren Kolonnenendes angebracht. Gegen die Umgebung war die Strippkolonne thermisch isoliert. Oberhalb des letzten Dual-Flow-Bodens war ein Kaminboden als Sammelboden angebracht. Die Oberkante der Kamine dieses Sammelbodens befand sich 29525 mm oberhalb des unteren Kolonnenendes. Die Kamine waren bedacht und wiesen einen Innendurchmesser von 316,7 mm und eine Höhe (gerechnet bis Überlaufhöhe ohne Hut) von 1030 mm auf. Ihre Gesamtzahl betrug 12 und war über den Kaminboden gleichförmig verteilt. Der Sammelboden war einfachwandig mit 2° Gefälle nach Außen und mit seitlichem Abzug und Abzugsstutzen (DN - 200) gestaltet. Der freie Gasquerschnitt betrug ca. 30 %. 4940 mm oberhalb der Kaminoberkante (ohne Hut gerechnet) waren durch die Kolonnenwand radial in die Kolonne sechs Rohre eingeführt, deren Innendurchmesser 82 mm und deren Wanddicke 2,6 mm betrug. Über den Umfang der Kolonne waren die Einführstellen der Rohre äquidistant verteilt (Einschlusswinkel zweier benachbarter Rohre = 60°).

In einer Entfernung von 500 mm von der Innenwand der Kolonne waren fünf der sechs Rohre nach unten gekrümmt und endeten in einer kreisförmigen Düsenöffnung mit einem Innendurchmesser von 2,5 Zoll.

Das sechste Rohr hatte eine von der Kolonneninnenwand radial ins Kolonneninnere reichende Länge von 800 mm. Im Abstand von 500 mm von der Kolonneninnenwand wies es eine nach unten weisende kreisförmige Düsenöffnung mit einem Innendurchmesser von ebenfalls 2,5 Zoll auf. Am Längenende wies dieses Rohr eine zusätzliche kreisförmige Düsenöffnung mit einem Innendurchmesser von 1 ¼ Zoll auf. Der Zentralstrahl des zugehörigen Sprühkegels wies eine nach oben gerichtete Vektorkomponente auf und schloss mit der Vertikalen zum Kolonnenquerschnitt einen Winkel von 15° ein. Über eine außerhalb der Kolonne angebrachte Ringleitung, an die die sechs Rohre angeschlossen waren, wurden die sechs Rohre mit der Flüssigkeit zur Direktkühlung des die Acrylsäure frei gestrippt enthaltenden Gases, das durch den Kaminboden strömte, versorgt und diese Flüssigkeit ins Kolonneninnere versprüht. Durch die Direktkühlung bildete sich überwiegend aus Acrylsäure bestehendes Kondensat, das auf dem Kaminboden gesammelt wurde. Die Länge der Strippkolonne insgesamt (von ihrem unteren Ende bis zu ihrem oberen Gasauslaß) betrug 35260 mm.

Die Zufuhr der 1600 kg/h an der Kondensationskolonne entnommener Sumpfflüssigkeit erfolgte auf den achten (8.) Dual-Flow-Boden von unten.

Dem Sumpf der Strippkolonne wurden 569979 kg/h an Sumpfflüssigkeit entnommen, deren Temperatur 160 °C betrug, und die folgende Gehalte aufwies (nachfolgend als "Strippsumpfflüssigkeit" bezeichnet):

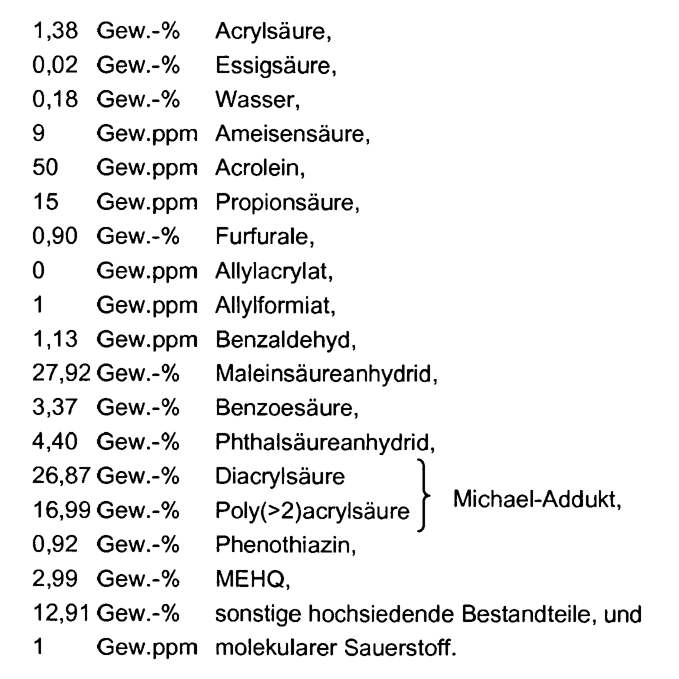

Mittels einer Pumpe (mit doppelt wirkender Gleitringdichtung gemäß der DE-A 10228859, wobei als Sperrflüssigkeit ein Wasser/Glykol-Gemisch verwendet wurde) wurde die entnommene Strippsumpfflüssigkeit in einen Dreistromrohrbündelwärmeübertrager gepumpt, durch dessen Rohre sie strömte (Sekundärseite). Der Außenrohrdurchmesser betrug 38 mm, die Wanddicke der Rohre war 2 mm. Die Länge der Rohre war 4800 mm und die Gesamtzahl der Rohre betrug 234 (jeweils 78 Rohre für eine Strömungsrichtung). Die Rohrteilung war 48 mm (30°-Teilung). Durch 9 zwischen den Rohrböden (in denen die Austauscherrohre befestigt waren) angebrachte Umlenkscheiben (Scheibendicke: jeweils 5 mm) war der die Wärmeübertragerrohre umgebende zylindrische Raum (Primärseite) in 10 Längsabschnitte (Segmente) unterteilt. Alle 9 Umlenkscheiben waren im Prinzip kreisförmig. Der Kreisdurchmesser betrug 859 mm. An jeder der kreisförmigen Umlenkscheiben war jedoch ein halbmondförmiges Kreissegment abgeschnitten, dessen Fläche 35,8 % der Gesamtfläche betrug, so dass ein entsprechender Durchtritt für den Wasserdampf entstand, wobei diese Durchtritte aufeinanderfolgend alternierend einander gegenüberliegend angebracht waren (im übrigen waren die Umlenkbleche an der Behälterwand abdichtend befestigt; dort wo Wärmeübertragerrohre auf die Umlenkbleche stießen, waren entsprechende Bohrungen in den Umlenkblechen). Dem die Wärmeübertragerrohre umgebenden Raum wurden 1600 kg/h an Wasserdampf zugeführt, dessen Temperatur am Eingang 212 °C und dessen Eingangsdruck 20 bar war. Der Eintritt von Wasserdampf und Strippsumpfflüssigkeit in den Dreistromrohrbündelwärmeübertrager befand sich auf derselben Seite des Wärmeübertragers.

Von der aus dem Dreistromrohrbündelwärmeübertrager mit einer Temperatur von 165°C ausströmenden erhitzten Strippsumpfflüssigkeit wurden nur 569408 kg/h in die Strippkolonne rückgeführt. Die anderen 570,9 kg/h davon wurden entgast, und mit 79,1 kg/h Methanol verdünnt der Rückstandsverbrennung zugeführt. Zwischen dem Austritt der erhitzten Strippsumpfflüssigkeit aus dem Dreistromrohrbündelwärmeübertrager und der Wiedereintrittstelle der erhitzten Strippsumpfflüssigkeit in die Strippkolonne war eine Lochblende angebracht. Diese gewährleistete in Strömungsrichtung vor der Lochblende einen Arbeitsdruck von 3 bar, während der Arbeitsdruck innerhalb der Strippkolonnen unmittelbar oberhalb des Sumpfflüssigkeitsspiegels 1,75 bar betrug. Der Wiedereintritt der erhitzten Strippsumpfflüssigkeit war als in die Strippkolonnenquerschnittsmitte gezogenes koaxiales Doppelrohr ausgeführt, wo es nach unten gekrümmt auf den Spiegel der Sumpfflüssigkeit zeigte und kurz oberhalb der Spiegelfläche endete. Im äußeren Ring des koaxialen Doppelrohres wurde die überhitzte Sumpfflüssigkeit geführt, im Kern des koaxialen Doppelrohres wurde gleichzeitig das Strippgas zudosiert. Bei diesem handelte es sich um vom Kolonnenkopf der Kondensationskolonne entnommenes Restgas, das (gemeinsam mit Kreisgas) auf einen Arbeitsdruck von 2,9 bar verdichtet (mit Hilfe eines mehrstufigen Radialverdichters) worden war und eine Temperatur von 160 °C aufwies. Sein Mengenstrom lag bei 28869,9 kg/h. Es wies folgende Gehalte auf:

| | | |
|---|---|---|
| 0,28 | Gew.-% | Acrylsäure, |
| 0,10 | Gew.-% | Essigsäure, |
| 3,06 | Gew.-% | Wasser, |
| 62 | Gew.ppm | Ameisensäure, |
| 0,17 | Gew.-% | Acrolein, |
| 3 | Gew.ppm | Propionsäure, |
| 2 | Gew.ppm | Furfurale, |
| 13 | Gew.ppm | Allylformiat, |
| 4,72 | Gew.-% | molekularen Sauerstoff, |
| 2,11 | Gew.-% | Kohlendioxid, |
| 0,69 | Gew.-% | Kohlenmonoxid, |
| 0,65 | Gew.-% | Propan, |
| 0,32 | Gew.-% | Propylen, und |
| 87,90 | Gew.-% | molekularen Stickstoff. |

Dem Sammelboden (Kaminboden) wurden 147877,1 kg/h an Kondensat entnommen, das eine Temperatur von 65 °C aufwies.

Die verbliebene Teilmenge der 2600 kg/h an der Kondensationskolonne entnommener Kondensationssumpfflüssigkeit, die sich auf 1000 kg/h belief, wurde mit vorgenannter entnommener Kondensatmenge zusammengeführt. 13173,5 kg/h des dabei resultierenden flüssigen Gemischs, das eine Temperatur von geringfügig oberhalb 65 °C aufwies, wurden unterhalb des Kaminbodens, aber oberhalb des obersten Dual-Flow-Bodens als Rücklaufflüssigkeit in die Strippkolonne rückgeführt.

Zum Zweck dieser Rückführung befand sich 770 mm oberhalb des obersten Dual-Flow-Bodens ein zu einem geschlossenen Kreis ausgeführtes Verteilerrohr (in die Kolonne zentriert sowie waagrecht, d. h., parallel zum Kolonnenquerschnitt, eingebracht) dem die Rücklaufflüssigkeit zugeführt wurde. Der Kreisinnendurchmesser betrug 1870 mm. Der Rohraußendurchmesser betrug 33,7 mm und der Rohrinnendurchmesser war 25 mm.

Das kreisförmig ausgeführte Verteilerrohr wies 21 Lochöffnungen auf, deren Innendurchmesser 5 mm betrug. Jede zweite dieser Öffnungen war auf die Unterseite des Kaminbodens gerichtet, um diesen mit Rücklaufflüssigkeit feucht zu halten. Der Zentralstrahl aus der anderen Hälfte der Öffnungen wies in einem Winkel von 45° (zur Senkrechten auf den Kolonnenquerschnitt) hälftig nach unten in die Kolonnenmitte sowie hälftig nach unten zur Kolonnenwand.

Zusätzlich waren am Umfang des kreisförmig ausgeführten Verteilerrohres drei über den Umfang gleichmäßig verteilte (Einschlusswinkel 120°) und radial nach außen weisende Präzisionsstrahlrohre (Länge = 200 mm, Außendurchmesser = 6 mm, Innendurchmesser = 4 mm) angebracht. Der Austritt aus den Präzisionsstrahlrohren war auf Kugelhähne gerichtet, über die der Kolonne Waschflüssigkeit zugeführt werden konnte. Über die Lochöffnungen und die Strahlrohre wurde die Rücklaufflüssigkeit dem trennwirksamen Teil zugeführt.

Sie wies folgende Gehalte auf:

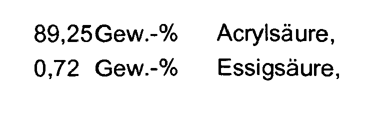

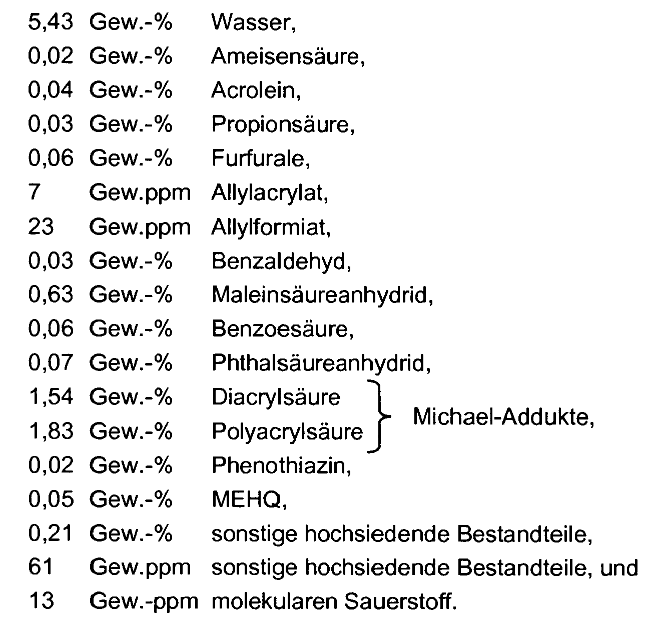

Die restlichen 135703,6 kg/h des eine Temperatur von 60 °C aufweisenden flüssigen Gemischs wurden durch einen mit Wasser (Eintrittstemperatur = 20 °C) gekühlten (im Gegenstrom) Spiralwärmeaustauscher geführt, und dabei auf 32 °C abgekühlt. Zum Zweck der Direktkühlung des durch den Kaminboden in der Kolonne aufsteigenden, mit Acrylsäure beladenen, Strippgases wurde die Gesamtmenge der so indirekt abgekühlten flüssigen Gemischmenge über den beschriebenen doppelten Ringverteiler in die Strippkolonne versprüht.

Am Kopf der Kolonne wurden 30899,0 kg/h an mit Acrylsäure beladenem Strippgas herausgeführt, das einen Druck von 1,6 bar und eine Temperatur von 60 °C sowie die nachfolgenden Gehalte aufwies:

| | | |
|---|---|---|
| 6,72 | Gew.-% | Acrylsäure, |
| 0,11 | Gew.-% | Essigsäure, |
| 2,93 | Gew.-% | Wasser, |
| 66 | Gew.ppm | Ameisensäure, |
| 1 | Gew.ppm | Formaldehyd, |
| 0,16 | Gew.-% | Acrolein, |
| 22 | Gew.ppm | Propionsäure, |
| 36 | Gew.ppm | Furfurale, |
| 1 | Gew.ppm | Allylacrylat, |
| 12 | Gew.ppm | Allylformiat, |
| 10 | Gew.ppm | Benzaldehyd, |
| 0,01 | Gew.-% | Maleinsäureanhydrid, |
| 4,41 | Gew.-% | molekularen Sauerstoff, |
| 1,98 | Gew.-% | Kohlendioxid, |
| 0,65 | Gew.-% | Kohlenmonoxid, |
| 0,60 | Gew.-% | Propan, |
| 0,29 | Gew.-% | Propylen, und |
| 82,13 | Gew.-% | molekularen Stickstoff. |

Es wurde in seiner Gesamtmenge in den Sumpfraum der Kondensationskolonne rückgeführt (nicht getaucht).

Nach einer Betriebsdauer von 4 Tagen musste der vorstehend beschriebene Betrieb aufgrund des Ausmaßes des sich in den Rohren des Rohrbündelwärmeübertragers einstellenden Fouling unterbrochen werden, um die Wärmeübertragerrohre von den Feststoffabscheidungen auf ihrer inneren Oberfläche zu reinigen.

### 2. Vergleichsbeispiel

Das erste Vergleichsbeispiel wurde wiederholt, die der Strippkolonne entnommene Strippsumpfflüssigkeit enthielt jedoch, bezogen auf ihr Gewicht, 1 Gew.-% Komad^{®} 313 der Fa. Mol (Ungarn) zugesetzt.

Die Betriebsdauer konnte dadurch auf 13 Tage verlängert werden.

### 3. Vergleichsbeispiel

Das erste Vergleichsbeispiel wird wiederholt, die der Strippkolonne entnommene Strippsumpfflüssigkeit enthält jedoch, bezogen auf ihr Gewicht, 1 Gew.-% eines Propylenoxid/Ethylenoxid-Blockpolymeren [(EO)ₓ(PO)₅₆(EO)_{y}, x + y = 8] gemäß EP-A 1 062 197 zugesetzt.
Die Betriebsdauer kann dadurch auf 14 Tage verlängert werden.

### 1. Beispiel

Das erste Vergleichsbeispiel wird wiederholt, die der Strippkolonne entnommene Strippsumpfflüssigkeit enthält jedoch, bezogen auf ihr Gewicht, 1 Gew.-% der Wirkverbindung Pentamethyldiethylentriamin zugesetzt:

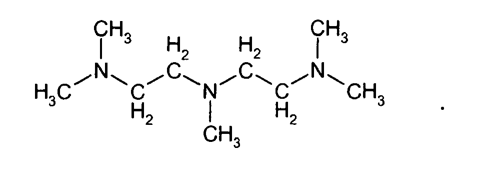

Die Betriebsdauer kann dadurch auf 29 Tage verlängert werden.

### 2. Beispiel

Wie das erste Beispiel, die Wirkverbindung ist jedoch N,N,N',N'-Tetramethyl-1,3-propandiamin:

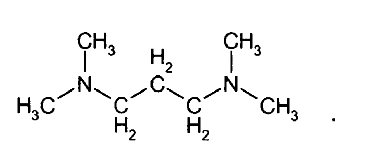

Die Betriebsdauer kann auf 29 Tage verlängert werden.

### 3. Beispiel

Wie das erste Beispiel, die Wirkverbindung ist jedoch Triethylamin:

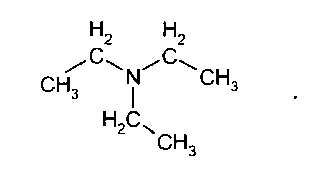

Die Betriebsdauer kann auf 28 Tage verlängert werden.

### 4. Beispiel

Wie das erste Beispiel, die Wirkverbindung ist jedoch N-Ethyl-N,N-diisopropylamin:

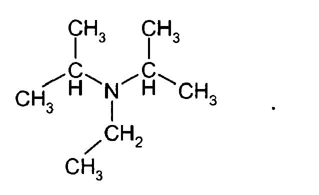

Die Betriebsdauer kann auf 28 Tage verlängert werden.

### 5. Beispiel

Wie das erste Beispiel, die Wirkverbindung ist jedoch N,N,N',N'-Tetramethylhexandiamin:

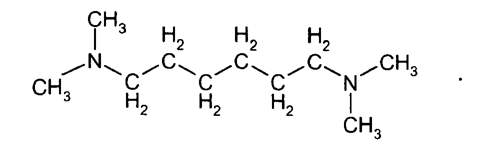

Die Betriebsdauer kann auf 27 Tage verlängert werden.

### 6. Beispiel

Wie das erste Beispiel, die Wirkverbindung ist jedoch Bis(2-dimethylaminoethyl)ether:

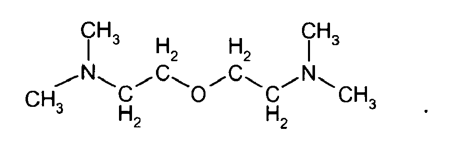

Die Betriebsdauer kann auf 27 Tage verlängert werden.

### 7. Beispiel

Wie das erste Beispiel, die Wirkverbindung ist jedoch 1-Methylimidazol:

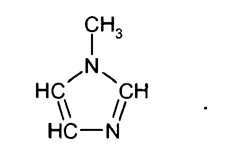

Die Betriebsdauer kann auf 26 Tage verlängert werden.

### 8. Beispiel

Wie das erste Beispiel, die Wirkverbindung ist jedoch N,N-Dimethylcyclohexylamin. Die Betriebsdauer kann auf 25 Tage verlängert werden.

### 9. Beispiel

Wie das erste Beispiel, die Wirkverbindung ist jedoch Tetramethylammoniumacetat. Die Betriebsdauer kann auf 29 Tage verlängert werden.

### 10. Beispiel

Wie das erste Beispiel, die Wirkverbindung ist jedoch 1-Ethyl-3-methyl-imidazoliumacrylat. Die Betriebsdauer kann auf 29 Tage verlängert werden.

### 11. Beispiel

Wie das erste Beispiel, die Wirkverbindung ist jedoch 1-Ethyl-3-methylimidazoliumacetat. Die Betriebsdauer kann auf 29 Tage verlängert werden.

### 12. Beispiel

Wie das erste Beispiel, die Wirkverbindung ist jedoch 1-Butyl-3-methylimidazoliumacetat. Die Betriebsdauer kann auf 28 Tage verlängert werden.

### 13. Beispiel

Wie das erste Beispiel, die Wirkverbindung ist jedoch Tetramethylammoniumhydroxid. Die Betriebsdauer kann auf 28 Tage verlängert werden.

### 14. Beispiel

Wie das erste Beispiel, die Wirkverbindung ist jedoch Tetramethylammoniumchlorid. Die Betriebsdauer kann auf 27 Tage verlängert werden.

### 15. Beispiel

Wie das erste Beispiel, die Wirkverbindung ist jedoch 1-Ethyl-3-methylimidazolium-chlorid. Die Betriebsdauer kann auf 26 Tage verlängert werden.

### 16. Beispiel

Wie das erste Beispiel, zusätzlich zum Pentamethyldiethylentriamin enthält die Strippsumpfflüssigkeit jedoch noch 0,3 Gew.-% Komad^{®} zugesetzt. Die Betriebsdauer kann dadurch auf 34 Tage verlängert werden.

Die erfindungsgemäß Verfahrensweise gewährleistet eine erhöhte Raum-ZeitAusbeute an abgetrennter Acrylsäure.

US Provisional Patent Application No. 60/871,529, eingereicht am 22.12.06 ist eingefügt in die vorliegende Anmeldung durch Literaturhinweis. Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

## Patentansprüche

1. Verfahren zur Übertragung von Wärme auf ein wenigstens ein (Meth)acryl-monomeres enthaltendes flüssiges Gemisch mit Hilfe eines indirekten Wärmeaustauschers, der auf seiner Primärseite von einem fluiden Wärmeträger und auf seiner Sekundärseite gleichzeitig von dem wenigstens ein (Meth)acrylmono-meres enthaltenden flüssigen Gemisch durchströmt wird, **dadurch gekennzeichnet, dass** das wenigstens ein (Meth)acrylmonomeres enthaltende flüssige Gemisch wenigstens eine von (Meth)acrylmonomeren verschiedene Wirkverbindung aus der Gruppe bestehend aus tertiären Aminen, den Salzen gebildet aus einem tertiären Amin und einer Brönsted-Säure, sowie quartären Ammoniumverbindungen mit der Maßgabe zugesetzt enthält, dass von den tertiären und quartären Stickstoffatomen in der wenigstens einen Wirkverbindung keines eine Phenylgruppe, aber wenigstens eine Teilmenge wenigstens eine Alkylgruppe trägt.

2. Verfahren nach Anspruch 1, **dadurch gekennzeichnet, dass** das wenigstens eine (Meth)acrylmonomere Acrylsäure und/oder Methacrylsäure ist.

3. Verfahren nach Anspruch 1, **dadurch gekennzeichnet, dass** das wenigstens eine (Meth)acrylmonomere wenigstens ein Monomeres aus der Gruppe bestehend aus Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, iso-Butylmethacrylat, n-Butylmethacrylat, tert.-Butylacrylat, tert.-Butylmethacrylat, Ethylacrylat, Ethylmethacrylat, 2-Ethylhexylacrylat und 2-Ethylhexylmethacrylat ist.

4. Verfahren nach einem der Ansprüche 1 bis 3, **dadurch gekennzeichnet, dass** die wenigstens eine Wirkverbindung ein tertiäres Amin der allgemeinen Formel 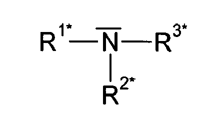 mit
R¹*, R²* und R³* unabhängig voneinander eine 1 bis 8 C-Atome aufweisende Al- kylgruppe, oder eine 1 bis 8 C-Atome aufweisende Alkylgruppe, bei der ein oder mehrere Wasserstoffatome durch wenigstens eine der Gruppen -OH, -NH₂, -NOCH₃ und -N(CH₃)₂ ersetzt und/oder die Kohlenstoffkette wenigstens einmal durch ein Sauerstoffatom unterbrochen ist, oder eine 1 bis 8 C-Atome aufweisende Cycloalkylgruppe bei der ein oder mehr Wasser- stoffatome durch wenigstens eine der Gruppen -OH, -NH₂, -NHCH₃ und -N(CH₃)₂ ersetzt und/oder die cyclische Kohlen- stoffkette wenigstens einmal durch ein Sauerstoffatom unter- brochen ist,
oder das Salz aus einem solchen tertiären Amin und einer Brönsted-Säure ist.

5. Verfahren nach einem der Ansprüche 1 bis 3, **dadurch gekennzeichnet, dass** die wenigstens eine Wirkverbindung ein Derivat des 1,3-Diazols ist, das sich von diesem **dadurch** ableitet, dass der Wasserstoff am Stickstoff in der 1-Stellung durch eine Alkylgruppe R⁴ mit 1 bis 8 C-Atomen ersetzt und/oder der Stickstoff des 1,3-Diazols in der 3-Stellung mit einer Alkylgruppe R⁵ mit 1 bis 8 C-Atomen alkyliert ist.

6. Verfahren nach einem der Ansprüche 1 bis 3, **dadurch gekennzeichnet, dass** die wenigstens eine Wirkverbindung das Salz eines quartären Ammoniumions ist, wobei das quartäre Ammoniumion ein solches der allgemeinen Formel 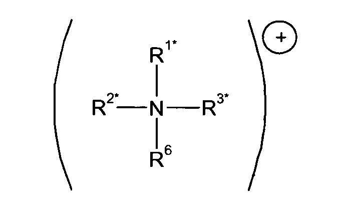 mit
R¹*, R²* und R³* unabhängig voneinander und unabhängig von R⁶ eine 1 bis 8 C-Atome aufweisende Alkylgruppe, oder eine 1 bis 8 C-Atome aufweisende Alkylgruppe, bei der ein oder mehrere Wasser- stoffatome durch wenigstens eine der Gruppen -OH, -NH₂, -NHCH₃ und -N(CH₃)₂ ersetzt und/oder die Kohlenstoffkette wenigstens einmal durch ein Sauerstoffatom unterbrochen ist, oder eine 1 bis 8 C-Atome aufweisende Cycloalkylgruppe, oder eine 1 bis 8 C-Atome aufweisende Cycloalkylgruppe, bei der ein oder mehr Wasserstoffatome durch wenigstens eine der Gruppen -OH, -NH₂, -NHCH₃ und -N(CH₃)₂ ersetzt und/oder die cyclische Kohlenstoffkette wenigstens einmal durch ein Sauerstoffatom unterbrochen ist, und
R⁶ unabhängig von R¹*, R²* und R³* eine 1 bis 8 C-Atome aufwei- sende Alkylgruppe,
ist.

7. Verfahren nach einem der Ansprüche 1 bis 6, **dadurch gekennzeichnet, dass** das Molgewicht der wenigstens einen Wirkverbindung ≤ 600 g beträgt.

8. Verfahren nach einem der Ansprüche 1 bis 7, **dadurch gekennzeichnet, dass** das flüssige Gemisch 0,01 bis 10 Gew.-% seines Gewichtes von der wenigstens einen Wirkverbindung zugesetzt enthält.

9. Verfahren nach einem der Ansprüche 1 bis 8, **dadurch gekennzeichnet, dass** das flüssige Gemisch zu ≥ 0,5 Gew.-% seines Gewichtes von dem wenigstens einen (Meth)acrylmonomeren enthält.

10. Verfahren nach einem der Ansprüche 1 bis 9, **dadurch gekennzeichnet, dass** die Temperatur, mit der das flüssige Gemisch den indirekten Wärmeaustauscher verlässt, 50 bis 350 °C beträgt.

11. Verfahren nach einem der Ansprüche 1 bis 10, **dadurch gekennzeichnet, dass** der indirekte Wärmeaustauscher ein Rohrbündelwärmeüberträger ist.

12. Verfahren nach einem der Ansprüche 1 bis 11, **dadurch gekennzeichnet, dass** der fluide Wärmeträger Wasserdampf ist.

13. Verfahren nach einem der Ansprüche 1 bis 12, **dadurch gekennzeichnet, dass** das flüssige Gemisch aus dem Sumpf einer trennwirksame Einbauten enthaltenden Trennkolonne entnommen wurde, in die im Rahmen eines thermischen Trennverfahrens wenigstens ein wenigstens ein (Meth)acrylmonomeres enthaltender Stoffstrom zugeführt und wenigstens ein von diesem verschiedener wenigstens ein (Meth)acrylmonomeres enthaltender Stoffstrom entnommen wird.

14. Verfahren nach Anspruch 13, **dadurch gekennzeichnet, dass** das flüssige Gemisch nach dem Verlassen des indirekten Wärmeaustauschers in die Trennkolonne rückgeführt wird.

15. Verfahren nach Anspruch 13 oder 14, **dadurch gekennzeichnet, dass** der wenigstens eine zugeführte Stoffstrom das Acrylsäure enthaltende Sumpfprodukt einer fraktionierenden Kondensation des Produktgasgemischs einer heterogen katalysierten Gasphasen-Partialoxidation einer C₃-Vorläuferverbindung zu Acrylsäure in einer Kondensationskolonne und das thermische Trennverfahren die Strippung der Acrylsäure aus diesem Sumpfprodukt ist.

## Claims

1. A process for transferring heat to a liquid mixture comprising at least one (meth)acrylic monomer with the aid of an indirect heat exchanger which is flowed through on its primary side by a liquid heat carrier and on its secondary side simultaneously by the liquid mixture comprising at least one (meth)acrylic monomer, wherein the liquid mixture comprising at least one (meth)acrylic monomer comprises at least one added active compound other than (meth)acrylic monomers from the group consisting of tertiary amines, the salts formed from a tertiary amine and a Brønsted acid, and quaternary ammonium compounds, with the proviso that none of the tertiary and quaternary nitrogen atoms in the at least one active compound bears a phenyl group but at least some bear at least one alkyl group.

2. The process according to claim 1, wherein the at least one (meth)acrylic monomer is acrylic acid and/or methacrylic acid.

3. The process according to claim 1, wherein the at least one (meth)acrylic monomer is at least one monomer from the group consisting of hydroxyethyl acrylate, hydroxyethyl methacrylate, hydroxypropyl acrylate, hydroxypropyl methacrylate, glycidyl acrylate, glycidyl methacrylate, methyl acrylate, methyl methacrylate, n-butyl acrylate, isobutyl acrylate, isobutyl methacrylate, n-butyl methacrylate, tert-butyl acrylate, tert-butyl methacrylate, ethyl acrylate, ethyl methacrylate, 2-ethylhexyl acrylate and 2-ethylhexyl methacrylate.

4. The process according to any of claims 1 to 3, wherein the at least one active compound is a tertiary amine of the general formula 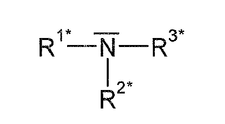 where
R¹*, R²* and R³* are each independently an alkyl group which has from 1 to 8 carbon atoms, or an alkyl group which has from 1 to 8 carbon atoms and in which one or more hydrogen atoms are replaced by at least one of the groups -OH, -NH₂, -NHCH₃ and -N(CH₃)₂ and/or the carbon chain is interrupted at least once by an oxygen atom, or a cycloalkyl group which has from 1 to 8 carbon atoms and in which one or more hydrogen atoms are replaced by at least one of the groups -OH, -NH₂, -NHCH₃ and -N(CH₃)₂ and/or the cyclic carbon chain is interrupted at least once by an oxygen atom,
or is the salt of such a tertiary amine and a Brønsted acid.

5. The process according to any of claims 1 to 3, wherein the at least one active compound is a derivative of 1,3-diazole which derives therefrom in that the hydrogen on the nitrogen in the 1-position has been replaced by an alkyl group R⁴ having from 1 to 8 carbon atoms and/or the nitrogen of the 1,3-diazole in the 3-position has been alkylated with an alkyl group R⁵ having from 1 to 8 carbon atoms.

6. The process according to any of claims 1 to 3, wherein the at least one active compound is the salt of a quaternary ammonium ion, where the quaternary ammonium ion is one of the general formula 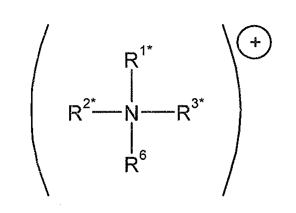 where
R¹*, R²* and R³* are each independently, and independently of R⁶, an alkyl group which has from 1 to 8 carbon atoms, or an alkyl group which has from 1 to 8 carbon atoms and in which one or more hydrogen atoms are replaced by at least one of the groups -OH, -NH₂, -NHCH₃ and -N(CH³)₂ and/or the carbon chain is interrupted at least once by an oxygen atom, or a cycloalkyl group which has from 1 to 8 carbon atoms, or a cycloalkyl group which has from 1 to 8 carbon atoms and in which one or more hydrogen atoms are replaced by at least one of the groups -OH, -NH₂, -NHCH₃ and -N(CH₃)₂ and/or the cyclic carbon chain
R⁶, is interrupted at least once by an oxygen atom, and independently of R¹*, R²* and R³*, is an alkyl group having from 1 to 8 carbon atoms.

7. The process according to any of claims 1 to 6, wherein the molar mass of the at least one active compound is ≤ 600 g.

8. The process according to any of claims 1 to 7, wherein the liquid mixture comprises an addition of from 0.01 to 10% by weight of its weight of the at least one active compound.

9. The process according to any of claims 1 to 8, wherein the liquid mixture comprises ≥ 0.5% by weight of its weight of the at least one (meth)acrylic monomer.

10. The process according to any of claims 1 to 9, wherein the temperature with which the liquid mixture leaves the indirect heat exchanger is from 50 to 350°C.

11. The process according to any of claims 1 to 10, wherein the indirect heat exchanger is a tube bundle heat transferer.

12. The process according to any of claims 1 to 11, wherein the fluid heat carrier is steam.

13. The process according to any of claims 1 to 12, wherein the liquid mixture has been withdrawn from the bottom of a separating column comprising separating internals, into which, in a thermal separating process, at least one stream comprising at least one (meth)acrylic monomer is fed and at least one stream which differs therefrom and comprises at least one (meth)acrylic monomer is withdrawn.

14. The process according to claim 13, wherein the liquid mixture, after leaving the indirect heat exchanger, is recycled into the separating column.

15. The process according to claim 13 or 14, wherein the at least one stream fed in is the acrylic acid-comprising bottom product of a fractional condensation of the product gas mixture of a heterogeneously catalyzed gas phase partial oxidation of a C₃ precursor compound to acrylic acid in a condensation column, and the thermal separating process is the stripping of the acrylic acid from this bottom product.

## Revendications

1. Procédé pour le transfert de chaleur à un mélange liquide contenant au moins un monomère (méth)acrylique, à l'aide d'un échangeur thermique indirect qui est traversé sur son côté primaire par un caloporteur fluide et sur son côté secondaire simultanément par ledit mélange liquide contenant au moins un monomère (méth)acrylique, **caractérisé en ce que** ledit mélange liquide contenant au moins un monomère (méth)acrylique contient ajouté au moins un composé actif différent de monomères (méth)acryliques, choisi dans le groupe constitué par des amines tertiaires, les sels formés à partir d'une amine tertiaire et d'un acide de Brönsted, ainsi que des composés ammonium quaternaire, étant entendu que dans ledit au moins un composé actif aucun des atomes d'azote tertiaires et quaternaires ne porte de groupe phényle, mais qu'au moins une quantité partielle porte au moins un groupe alkyle.

2. Procédé selon la revendication 1, **caractérisé en ce que** ledit au moins un monomère (méth)acrylique est l'acide acrylique et/ou l'acide méthacrylique.

3. Procédé selon la revendication 1, **caractérisé en ce que** ledit au moins un monomère (méth)acrylique est au moins un monomère choisi dans le groupe constitué par l'acrylate d'hydroxyéthyle, le méthacrylate d'hydroxyéthyle, l'acrylate d'hydroxypropyle, le méthacrylate d'hydroxypropyle, l'acrylate de glycidyle, le méthacrylate de glycidyle, l'acrylate de méthyle, le méthacrylate de méthyle, l'acrylate de n-butyle, l'acrylate d'isobutyle, le méthacrylate d'isobutyle, le méthacrylate de n-butyle, l'acrylate de tert-butyle, le méthacrylate de tert-butyle, l'acrylate d'éthyle, le méthacrylate d'éthyle, l'acrylate de 2-éthylhexyle et le méthacrylate de 2-éthylhexyle.

4. Procédé selon l'une quelconque des revendications 1 à 3, **caractérisé en ce que** ledit au moins un composé actif est une amine tertiaire de formule générale 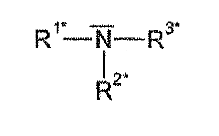 où
R¹*, R²* et R³* représentent, chacun indépendamment, un groupe alkyle comportant de 1 à 8 atomes de carbone, ou un groupe alkyle comportant de 1 à 8 atomes de carbone, dans lequel un ou plusieurs atomes d'hydrogène sont remplacés par au moins l'un des groupes -OH, -NH₂, -NHCH₃ et -N(CH₃)₂ et/ou la chaîne carbonée est interrompue au moins une fois par un atome d'oxygène, ou un groupe cycloalkyle comportant de 1 à 8 atomes de carbone, dans lequel un ou plusieurs atomes d'hydrogène sont remplacés par au moins l'un des groupes -OH, -NH₂, -NHCH₃ et -N(CH₃)₂ et/ou la chaîne carbonée cyclique est interrompue au moins une fois par un atome d'oxygène,
ou le sel d'une telle amine tertiaire et d'un acide de Brönsted.

5. Procédé selon l'une quelconque des revendications 1 à 3, **caractérisé en ce que** ledit au moins un composé actif est un dérivé du 1,3-diazole, qui dérive de celui-ci par le fait que l'atome d'hydrogène sur l'atome d'azote en la position 1 est remplacé par un groupe alkyle R⁴ ayant de 1 à 8 atomes de carbone et/ou l'atome d'azote du 1,3-diazole en la position 3 est alkylé par un groupe alkyle R⁵ ayant de 1 à 8 atomes de carbone.

6. Procédé selon l'une quelconque des revendications 1 à 3, **caractérisé en ce que** ledit au moins un composé actif est le sel d'un ion ammonium quaternaire, l'ion ammonium quaternaire étant un tel ion de formule générale 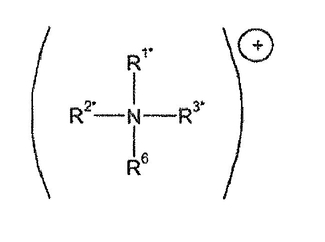 où
R¹*, R²* et R³* représentent, chacun indépendamment et indépendamment de R⁶, un groupe alkyle comportant de 1 à 8 atomes de carbone, ou un groupe alkyle comportant de 1 à 8 atomes de carbone, dans lequel un ou plusieurs atomes d'hydrogène sont remplacés par au moins l'un des groupes -OH, -NH₂, -NHCH₃ et -N(CH₃)₂ et/ou la chaîne carbonée est interrompue au moins une fois par un atome d'oxygène, ou un groupe cycloalkyle comportant de 1 à
R⁶ 8 atomes de carbone, ou un groupe cycloalkyle comportant de 1 à 8 atomes de carbone, dans lequel un ou plusieurs atomes d'hydrogène sont remplacés par au moins l'un des groupes -OH, -NH₂, -NHCH₃ et -N(CH₃)₂ et/ou la chaîne carbonée cyclique est interrompue au moins une fois par un atome d'oxygène, et représente, indépendamment de R¹*, R²* et R³*, un groupe alkyle comportant de 1 à 8 atomes de carbone.

7. Procédé selon l'une quelconque des revendications 1 à 6, **caractérisé en ce que** la masse moléculaire dudit au moins un composé actif est ≤ 600 g.

8. Procédé selon l'une quelconque des revendications 1 à 7, **caractérisé en ce que** le mélange liquide contient ajoutés 0,01 à 10 % en poids de son poids dudit au moins un composé actif.

9. Procédé selon l'une quelconque des revendications 1 à 8, **caractérisé en ce que** le mélange liquide contient à raison de ≥ 0,5 % % en poids de son poids ledit au moins un monomère (méth)acrylique.

10. Procédé selon l'une quelconque des revendications 1 à 9, **caractérisé en ce que** la température à laquelle le mélange liquide quitte l'échangeur thermique indirect est de 50 à 350 °C.

11. Procédé selon l'une quelconque des revendications 1 à 10, **caractérisé en ce que** l'échangeur thermique indirect est un échangeur de chaleur à faisceau de tubes.

12. Procédé selon l'une quelconque des revendications 1 à 11, **caractérisé en ce que** le caloporteur fluide est la vapeur d'eau.

13. Procédé selon l'une quelconque des revendications 1 à 12, **caractérisé en ce que** le mélange liquide a été prélevé du pied d'une colonne de séparation contenant des inserts facilitant la séparation, dans laquelle, dans le cadre d'un processus de séparation thermique, au moins un courant de substances contenant au moins un monomère (méth)acrylique est introduit et au moins un courant de substances contenant au moins un monomère (méth)acrylique différent de celui-ci est prélevé.

14. Procédé selon la revendication 13, **caractérisé en ce que** le mélange liquide, après avoir quitté l'échangeur thermique indirect, est renvoyé dans la colonne de séparation.

15. Procédé selon la revendication 13 ou 14, **caractérisé en ce que** ledit au moins un courant de substances est introduit le produit de pied, contenant de l'acide acrylique, d'une condensation avec fractionnement du mélange gazeux produit d'une oxydation partielle en phase gazeuse à catalyse hétérogène d'un composé précurseur en C₃ de l'acide acrylique dans une colonne de condensation et le procédé de séparation thermique est la séparation de l'acide acrylique à partir de ce produit de pied.
